# EUROPEAN PATENT APPLICATION

(11) **EP 4 751 731 A1**
(43) Date of publication of application: **03.06.2026**
(21) Application number: 24216609.8
(22) Date of filing: 29.11.2024
(51) Int. Cl.: A61K 39/00, C07K 16/28, A61K 39/395

(54) **COMBINATION OF A BISPECIFIC ANTIBODY CONSTRUCT, AN IMMUNE CHECKPOINT INHIBITOR AND A TAXANE**

(71) Applicant: Affimed GmbH, 68165 Mannheim (DE)
(72) Inventor: Pahl, Jens, 68165 Mannheim (DE); Stäble, Sina, 68165 Mannheim (DE); Morales-Espinosa, Daniela, 68165 Mannheim (DE); Dulat, Holger, 68165 Mannheim (DE)
(74) Representative: Schiweck Weinzierl Koch Patentanwälte Partnerschaft mbB

(57) **Abstract**

The invention relates to a bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor. The invention further relates to a composition comprising a taxane and a bispecific antibody construct that comprises at least one first binding domain capable of specifically binding CD16A and at least one second binding domain capable of specifically binding EGFR.

## Description

### FIELD OF THE INVENTION

The invention relates to a bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor. The invention further relates to a composition comprising a taxane and a bispecific antibody construct that comprises at least one first binding domain capable of specifically binding CD16A and at least one second binding domain capable of specifically binding EGFR. The present invention also relates to a combination comprising a taxane, a bispecific antibody construct that comprises at least one first binding domain capable of specifically binding CD16A and at least one second binding domain capable of specifically binding EGFR, and optionally an immune checkpoint inhibitor. It is also provided a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of M2 macrophages in a tumor microenvironment associated with the treatment of a tumor with a bispecific antibody construct and optionally an immune checkpoint inhibitor. Furthermore, the present invention provides a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of repolarizing M2 macrophages in the tumor microenvironment to M1 macrophages, wherein the M2 macrophages are associated with the treatment of a tumor with a bispecific antibody construct and optionally an immune checkpoint inhibitor.

### BACKGROUND

The treatment of non-small cell lung cancer (NSCLC) in the second and third lines is critical due to several limitations of current therapies. In particular traditional chemotherapy agents like docetaxel and pemetrexed have shown limited efficacy in improving overall survival and quality of life for patients in the second and third lines of treatment (Morabito BMC Medicine (2018) 16:24). The response rates are generally low, and the duration of response is often short. Moreover, many of the currently used chemotherapies are associated with significant toxicity and adverse effects, which can severely impact the patient's quality of life (Favaretto, Europ Oncol (2009) 5(1):38). This is particularly challenging for patients who have already undergone first-line treatments and may have compromised health. Another issue is that tumors often develop resistance to initial treatments, making subsequent therapies less effective (Davies 2017 ,PLOS ONE 0175679). This resistance can be due to various genetic and molecular changes in the tumor cells, which necessitates the need for new treatments that can overcome or bypass these resistance mechanisms. While newer therapies such as immunotherapy (e.g., nivolumab, pembrolizumab) and targeted therapies (e.g., EGFR inhibitors) have shown promise, they are not effective for all patients. There is a significant portion of patients who do not respond to these treatments or who experience progression after an initial response. Given these challenges, there is a high medical need for the development of new and more effective treatments for NSCLC patients in the second and third lines of therapy. These treatments should ideally have better efficacy, lower toxicity, and the ability to overcome resistance mechanisms.

The inventors observed that in the course of administration of an NK-cell engager the number of CD163-positive cells in the tumor increased. CD163 is considered a selective marker for immunosuppressive myeloid immune cells in the tumor microenvironment including immunosuppressive macrophages of subtype M2. CD163-positive macrophages of subtype M2 are known for their immunosuppressive properties in the tumor microenvironment and are linked to tumor progression. Thus, immunosuppression is undesirable because it reduces the anti-tumor activity of NK cells and T cells including response to PD-1/PD-L1 immune checkpoint blockade. Thus, to maximize the outcome of NK cell-based immunotherapy, immunosuppression in the tumor microenvironment needs to be overcome.

Previously, it has been reported that docetaxel treatment in an experimental mouse model could reshape the tumor microenvironment towards a less immunosuppressive milieu. This was characterized by the shift from M2 towards M1 myeloid cells, resulting in enhanced anti-tumor cytotoxic T cell responses and pro-inflammatory cytokines (Kodumudi et al. Clin Cancer Res

(2010) 16 (18): 4583-4594). However, such primary observation has never been confirmed in any preclinical and clinical setting. So far docetaxel is also understood to have a negative impact on the immunologic composition of the tumor micro environment (TME) by inhibiting NK cell anti-tumor cytotoxicity (Markasz et al. Mol Cancer Ther. 2007 Feb;6(2):644-54; Tong e al. Am J Clin Oncol. 2000 Oct;23(5):463-72). Thus, the negative effect of docetaxel on NK cell anti-tumor activity and the induction of immunosuppressive features in the TME by AFM24 might be expected to reduce anti-tumor activity when taxane is combined with NK cell-based immunotherapy.

### SUMMARY OF THE INVENTION

The inventors used a taxane, e.g. docetaxel and were able to observe that the number of macrophages with subtype M2 decreased, while the number of macrophages with subtype M1 increased. Macrophages with subtype M1 are not considered immunosuppressive; instead, macrophages with subtype M1 are characterized by anti-tumoral functionality and promote anti-tumor immunity. In other words, the inventors were able to counteract the unexpected finding that the administration of an NK cell engager causes an increase in the number of macrophages of subtype M2 increases with the administration of a taxane, e.g. docetaxel, so that the increase in the immunosuppressive M2 macrophages decreased and the number of M1 macrophages increased.

In addition to an NK cell engager and a taxane, e.g. docetaxel, a PD-L1 antagonist, e.g. antibodies, can also be administered. It has been observed that an NK cell engager acts synergistically with PD-L1 antagonists (PCT/IB2024/055675).

Therefore, the present invention is based at least partly on the surprising finding that taxanes could revert the M2 polarization of macrophages to M1 polarization and thereby turning the macrophages from tumor promoting to tumor suppressive macrophages.

Thus, the invention relates in a first aspect to a bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.

In an embodiment the tumor to be treated is a solid tumor.

In a further embodiment the tumor is characterized by epidermal growth factor receptor (EGFR) overexpression on tumor cells.

In some embodiments, the tumor is an EGFR-positive tumor.

In some embodiments, the tumor is selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN).

In some embodiments, the tumor is lung cancer.

In some embodiments, the tumor is non-small cell lung cancer (NSCLC).

In some embodiments, the first binding domain binds to an epitope on CD16A which is C-terminal to the physiological Fcy receptor binding domain, said epitope preferably comprising Y158 of SEQ ID NO: 1.

In some embodiments, the first binding domain comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).

In some, embodiments, the first binding domain comprises
(i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
(ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
(iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
(iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
(v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
(vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
(vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
(viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
(ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55.

In some embodiments, the first binding domain comprises
(i) a VH region as depicted in SEQ ID NO: 14 and a VL region as depicted in SEQ ID NO: 15;
(ii) a VH region as depicted in SEQ ID NO: 22 and a VL region as depicted in SEQ ID NO: 23;
(iii) a VH region as depicted in SEQ ID NO: 30 and a VL region as depicted in SEQ ID NO: 31;
(iv) a VH region as depicted in SEQ ID NO: 44 and a VL region as depicted in SEQ ID NO: 45;
(v) a VH region as depicted in SEQ ID NO: 46 and a VL region as depicted in SEQ ID NO: 47;
(vi) a VH region as depicted in SEQ ID NO: 48 and a VL region as depicted in SEQ ID NO: 49;
(vii) a VH region as depicted in SEQ ID NO: 53 and a VL region as depicted in SEQ ID NO: 50;
(viii) a VH region as depicted in SEQ ID NO: 55 and a VL region as depicted in SEQ ID NO: 51; or
(ix) a VH region as depicted in SEQ ID NO: 52 and a VL region as depicted in SEQ ID NO: 53.

In some embodiments, the first binding domain is a variable domain (Fv), a single chain Fv (scFv), a Fab, a single chain diabody (scDb), a diabody (Db) or a double Fab.

In some embodiments, the second binding domain comprises a VH and a VL domain of an antibody.

In some embodiments, the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In some embodiments, the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62.

In some embodiments, the second binding domain comprises a VH region as depicted in SEQ ID NO: 61 and a VL region as depicted in SEQ ID NO: 62.

In some embodiments, the second binding domain is a variable domain (Fv), a single chain Fv (scFv), a Fab, a single chain diabody (scDb), a diabody (Db) or a double Fab.

In some embodiments, the bispecific antibody construct binds to a target cell and an immune effector cell simultaneously.

In some embodiments, the bispecific antibody construct further comprises a third domain comprising a half-life extension domain.

In some embodiments, said half-life extension domain comprises a CH2 domain, wherein the Fcy receptor binding domain is silenced.

In some embodiments, said half-life extension domain comprises a CH3 domain.

In some embodiments, the bispecific antibody construct comprises at least one hinge domain and a CH3 domain fused to a CH2 domain in an amino to carboxyl order in the order hinge domain - CH2 domain - CH3 domain.

In some embodiments, the antibody construct comprises at least two of the hinge domain - CH2 domain - CH3 domain elements.

In some embodiments, the second binding domain is fused to the C terminus of a CH3 domain and the first binding domain is fused to the N terminus of a hinge region.

In some embodiments, the antibody construct is bivalent for the first binding domain and bivalent for the second binding domain.

In some embodiments, (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In some embodiments, (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab; and (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and (c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In some embodiments, (a) the first binding domain comprises a VH region as depicted in SEQ ID NO: 14 and a VL region as depicted in SEQ ID NO: 15; and (b) the second binding domain comprises a VH region as depicted in SEQ ID NO: 61 and a VL region as depicted in SEQ ID NO: 62.

In some embodiments, (a) the first binding domain comprises a VH region as depicted in SEQ ID NO: 14 and a VL region as depicted in SEQ ID NO: 15, wherein said first binding domain is a Fab; and (b) the second binding domain comprises a VH region as depicted in SEQ ID NO: 61 and a VL region as depicted in SEQ ID NO: 62, wherein said second binding domain is a scFv; and (c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In some embodiments, the antibody construct comprises or consists of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.

In some embodiments, the antibody construct comprises or consists of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.

In some embodiments, the bispecific antibody construct is AFM24.

In some embodiments, the at least one first binding domain of the bispecific antibody construct comprises means for specifically binding CD16A and the at least one second binding domain of the bispecific antibody construct comprises means for specifically binding EGFR.

In some embodiments, the bispecific antibody construct comprises means for specifically binding CD16A and EGFR.

In some embodiments, the taxane is selected from the group consisting of docetaxel, paclitaxel, cabazitaxel.

In some embodiments, the taxane is docetaxel.

In some embodiments, the immune checkpoint inhibitor is a PD-L1 or PD-1 inhibitor.

In some embodiments, the immune checkpoint inhibitor is an anti-PD-1 antibody or an anti-PD-L1 antibody.

In some embodiments, the immune checkpoint inhibitor is an anti-PD-L1 antibody.

In some embodiments, the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301, wherein the immune checkpoint inhibitors nivolumab atezolizumab and pembrolizumab are preferred and wherein atezolizumab is most preferred.

In some embodiments, the immune checkpoint inhibitor is atezolizumab.

In some embodiments, the immune checkpoint inhibitor is an antibody comprising means for specifically binding PD-L1.

In some embodiments, the bispecific antibody construct is AFM24, the taxanes is docetaxel, and the immune checkpoint inhibitor is atezolizumab.

In some embodiments, the bispecific antibody construct is administered to the subject once every about 6 to about 8 days, or once every about 7 days.

In some embodiments, the bispecific antibody construct is administered at a dose in the range of about 300 to about 800 mg, about 350 to about 700 mg, about 400 to about 550 mg, about 420 to about 530 mg, about 450 to about 500 mg, or about 480 mg.

In some embodiments, the taxane is administered to the subject once every about 2 to about 4 weeks, once every about 3 weeks, once every about 19 to about 23 days, once every about 20 to about 22 days, or once every about 21 days.

In some embodiments, the taxane is administered at a dose in the range of about 60 to about 90 mg/m², about 70 to about 80 mg/m², about 72 to about 78 mg/m², or about 75 mg/m².

In some embodiments, the immune checkpoint inhibitor is administered to the subject once every about 2 to about 4 weeks, once every about 3 weeks, once every about 19 to about 23 days, once every about 20 to about 22 days, or once every about 21 days.

In some embodiments, the immune checkpoint inhibitor is administered at a dose in the range of about 1000 to about 1400 mg, about 1100 to about 1300 mg, about 1150 to about 1250 mg, or about 1200 mg.

In some embodiments, a cycle of administering consists of administering the bispecific antibody construct molecule 3 times, administering the taxane 1 time, and administering the immune checkpoint inhibitor 1 time, and treatment of the subject is carried out for at least one cycle.

In some embodiments, a cycle of administering is about 3 weeks.

In some embodiments, administering of the taxane and administering of the immune checkpoint inhibitor are separated by a time period of at least about 5 days, at least about 6 days or at least about 7 days.

In some embodiments, the taxane is administered in the first week of each cycle.

In some embodiments, a dose the taxane and a dose of the bispecific antibody construct are administered to the subject at the same time, or about the same time.

In some embodiments, a dose the taxane is concurrently administered with a dose of the bispecific antibody construct.

In some embodiments, a dose of the bispecific antibody molecule and/or a dose the taxane are administered as split-day dosing on two consecutive days.

In some embodiments, the first dose of the bispecific antibody molecule and the dose of taxane during the first cycle are administered as split-day dosing on two consecutive days.

In some embodiments, the immune checkpoint inhibitor is administered in the second week of each cycle.

In some embodiments, a dose the immune checkpoint inhibitor and a dose of the bispecific antibody construct are administered to the subject at the same time, or about the same time.

In some embodiments, a dose the immune checkpoint inhibitor is concurrently administered with a dose of the bispecific antibody construct

In some embodiments, a lead-in dose of the bispecific antibody construct is administered to the subject about 5 to about 8 days or about 6 to about 7 days prior to a first cycle of administration.

In some embodiments, the lead-in dose is in the range of about 400 to about 550 mg, about 420 to about 530 mg, about 450 to about 500 mg, or about 480 mg.

In some embodiments, the lead-in dose is administered as a split-dose on two consecutive days.

In some embodiments, the bispecific antibody construct, the taxane, and/or the immune checkpoint inhibitor are administered intravenously.

In some embodiments, administering is carried out for a period of time in the range of 2 weeks to 2 years, or 1 month to 18 months, or 3 months to 12 months.

In some embodiments, the method comprises assessing tumor regression after each cycle of treatment.

In some embodiments, the bispecific antibody construct, preferably AFM24, is administered to the subject on day 1, 8, and 15 of each 21-day cycle at a dose of 480 mg weekly; and the taxane, preferably docetaxel, is administered to the subject on day 1 of each 21-day cycle at a dose of 75 mg/m2 every 3 weeks (q3w); and the immune checkpoint inhibitor, preferably atezolizumab, is administered to the subject on day 8 of each 21-day cycle at a dose of 1200 mg q3w; wherein the bispecific antibody construct and the taxanes is administered to the subject on day 1 of the first and second cycle as a split day-dose on day 1 and 2 and wherein the treatment lasts up to 24 months or disease progression, optionally wherein a lead-in dose of the bispecific antibody construct, preferably AFM24, is administered to the subject as a split-day dose on days 7 and 6 prior to the first cycle in an amount of 480 mg.

In some embodiments, treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.

In some embodiments, treating reduces the number of M2 macrophages in the tumor microenvironment as compared to a treatment regimen comprising the administration of the bispecific antibody molecule and the immune checkpoint inhibitor, but without the administration of a taxane.

In some embodiments, treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a taxanes.

In some embodiments, treating repolarizes M2 macrophages in the tumor microenvironment to M1 macrophages, wherein the M2 macrophages are associated with the treatment of a tumor with the bispecific antibody construct.

In a second aspect, the invention relates to a pharmaceutical composition comprising a taxane and a bispecific antibody construct that comprises at least one first binding domain capable of specifically binding CD16A and at least one second binding domain capable of specifically binding EGFR.

In some embodiments, the taxane is present in an amount (by weight) that is in the range of about 5 to about 8 times lower than an amount of the bispecific antibody construct.

In some embodiments, the pharmaceutical composition is in liquid form and configured for IV administration.

In some embodiments, the pharmaceutical composition comprises one or more excipients.

In some embodiments, the pharmaceutical composition is for use in a method of treating a tumor in a subject.

In some embodiments, the use of the pharmaceutical composition is the use in a method of treating as defined in any one of the embodiments of the first aspect.

In a third aspect, the invention relates to a combination comprising a taxane, a bispecific antibody construct that comprises at least one first binding domain capable of specifically binding CD16A and at least one second binding domain capable of specifically binding EGFR, and optionally an immune checkpoint inhibitor.

In some embodiments, the combination is for use in a method of treating a tumor in a subj ect.

In some embodiments, the use of the combination is the use in a method of treating as defined in any one of the embodiments of the first aspect.

In a fourth aspect, the invention relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of M2 macrophages in a tumor microenvironment associated with the treatment of a tumor with a bispecific antibody construct and optionally an immune checkpoint inhibitor.

In some embodiments, the use of the taxane, bispecific antibody construct, or combination is the use in a method of treating as defined in any one of the embodiments of the first aspect.

In a fifth aspect, the invention relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor.

In some embodiments, the use of the taxane, bispecific antibody construct, or combination is the use in a method of treating as defined in any one of the embodiments of the first aspect.

In a sixth aspect, the invention relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of repolarizing M2 macrophages in the tumor microenvironment to M1 macrophages, wherein the M2 macrophages are associated with the treatment of a tumor with a bispecific antibody construct and optionally an immune checkpoint inhibitor.

In some embodiments, the use of the taxane, bispecific antibody construct, or combination is the use in a method of treating as defined in any one of the embodiments of the first aspect.

### BRIEF DESCRIPTION OF THE DRAWINGS

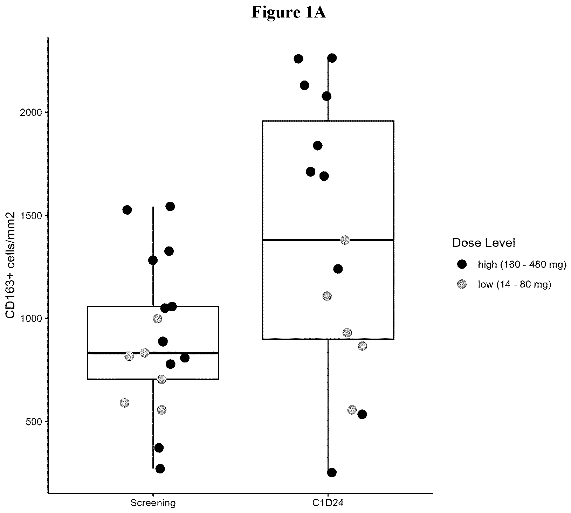

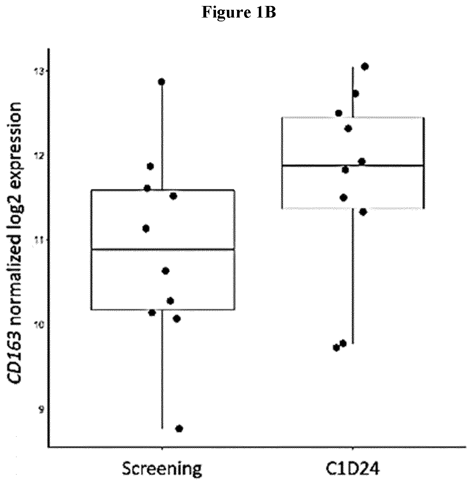

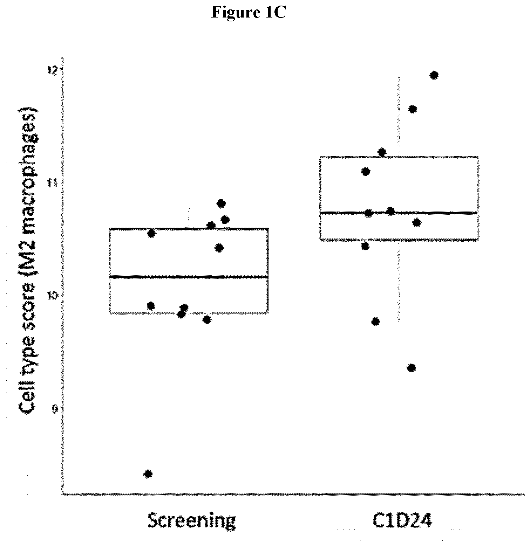

**Figure 1****: Analysis of tissue biopsies indicates a polarization of tumor-associated macrophages towards immune suppressive M2 macrophages in the tumor upon AFM24 treatment.**
**Figure 1****.A:** Quantification of CD163-positive cells (M2 macrophages) in paired biopsies from screening and at C1D24. Box plot showing the cell density (cells per mm³) of CD163-positive cells in tumor tissue determined by IHC. Dots represent individual patients where data from low dose (14 mg - 80 mg, n = 5) cohorts are depicted as light grey dots and data from high dose (160 mg - 480 mg, n = 10) cohorts are shown as black dots.
**Figure 1****.B:** Quantification of CD163 expression in paired biopsies from screening and at C1D24 of patients treated with AFM24 at doses higher than 160 mg. Biopsies have been analyzed by gene expression profiling using the Nanostring nCounter^{®} PanCancer Immune Profiling Panel and Tumor Signaling 360^{™} Panel, respectively. Boxplot showing the normalized log2 expression of *CD163* in paired tumor biopsies. Dots represent individual patients. (n = 10).
**Figure 1****.C:** Cell type score calculation for M2 macrophages in paired biopsies from screening and at C1D24 of patients treated with AFM24 at doses higher than 160 mg. Biopsies have been analyzed by gene expression profiling using the Nanostring nCounter^{®} PanCancer Immune Profiling Panel and Tumor Signaling 360^{™} Panel, respectively. The M2 macrophage cell type score is based on the following M2 macrophage specific marker genes: *CD163, CD68, CD84.* Boxplot showing the log2 cell type score for M2 macrophages in paired tumor biopsies. Dots represent individual patients. (n = 10).

### DETAILED DESCRIPTION

The inventors observed that in the case of administration of an NK-cell engager to a tumor in a patient, the number of macrophages of subtype M2 in the tumor or in the tumor environment increased. A marker for the subtype M2 of macrophages is CD163. However, macrophages of subtype M2 have an immunosuppressive effect. However, immunosuppression is undesirable because it affects the activity of NK cells. For the therapy with NK cells, one does not want to have an immunosuppressive environment.

The inventors surprisingly found that an addition of a taxane, e.g. docetaxel results in a decrease of the number of macrophages with subtype M2. Remarkably, at the same time the number of macrophages with subtype M1 increased.

Macrophages with subtype M1 do not have an immunosuppressive effect. In other words, the inventors were able to counteract the unexpected finding that the administration of an NK cell engager causes an increase in the number of macrophages of subtype M2 increases with the administration of a taxane, e.g. docetaxel, so that the increase in the immunosuppressive M2 macrophages decreased and the number of M1 macrophages increased.

In addition to an NK cell engager and a taxane, e.g. docetaxel, a PD-L1 antagonist, e.g. antibodies, can also be administered. It has been observed that an NK cell engager acts synergistically with PD-L1 antagonists.

In sum, the inventors found that the addition of a taxane counteracts AFM24-mediated increase in immunosuppressive factors of the tumor microenvironment that may restrict the maximal immune activating potential of AFM24 and of AFM24 in combination with an PD-L1 inhibitor. Furthermore, the addition of a taxane shows synergistic effects with both AFM24 and a PD-L1 inhibitor, sensitizing tumor cells to AFM24-ADCC. Thus, the addition of a taxane, e.g. docetaxel, may unleash the full anti-tumor potential of AFM24 in combination with a PD-L1 inhibitor and promote reactivation of cancer immunity cycle

Thus, the invention relates to a bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.

The invention also relates to a method of treating a tumor in a subject using a bispecific antibody construct, a taxane, and/or an immune checkpoint inhibitor, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.

The invention further relates to a use of a bispecific antibody construct, a taxane, and/or an immune checkpoint inhibitor for the manufacture of a medicament for the treatment of a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.

The inventive bispecific antibody construct, and/or taxane, and/or immune checkpoint inhibitor and compositions and combinations comprising the inventive bispecific antibody construct, and/or the taxane, and/or the immune checkpoint inhibitor are intended for use in a method of treating a tumor in a subject. The tumor is preferably a solid tumor. The tumor to be treated may be characterized by tumor cells which overexpress an epidermal growth factor receptor. Furthermore, the tumor to be treated may be an EGFR-positive tumor, e.g. such a tumor may comprise tumor cells which express or overexpress EGFR. Tumors to be treated with the inventive bispecific antibody construct, and/or taxane, and/or immune checkpoint inhibitor and compositions and combinations comprising the inventive bispecific antibody construct, and/or the taxane, and/or the immune checkpoint inhibitor may be selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN). Furthermore, the tumor to be treated may further be lung cancer. The tumor may be preferably a non-small cell lung cancer (NSCLC).

The tumor has a surrounding named tumor microenvironment comprising cancer cells, stromal tissue, extracellular matrix, and possibly blood vessels within and surrounding the tumor tissue. The tumor microenvironment comprises tumor-associated macrophages and T cells. Due to the immunosuppressive characteristics of the tumor microenvironment, for example by expression of checkpoint inhibitors, the macrophages are M2 polarized and anti-inflammatory, thus tumor-promoting. Furthermore, T cells within the immunosuppressive tumor environment are partly turned to Treg cells, which are tumor promoting and immunosuppressive by e.g. making CD8+ cell less effective.

As used herein, the terms "treatment," "treat," and "treating" refer to reversing, alleviating, ameliorating, delaying the onset of or inhibiting the progress of a tumor (as defined above) in a subject. For example, a treatment can result in a reduction in the amount of tumor cells or a reduced growth rate. Administration of a therapeutically effective amount of a compound as described herein for the treatment of a tumor will result in a reduction of the amount of tumor cells or a decreased growth rate, a reduction in risk or frequency of reoccurrence, a delay in reoccurrence, a reduction in metastasis, increased progression-free and overall survival, and/or decreased morbidity and mortality, among other things.

As used herein, the terms "alleviating" and "ameliorating" refer to any improvement of the disease state of a patient suffering from a tumor by the administration of the bispecific antibody construct and a taxane in accordance with the method of the invention. Such an improvement may also be seen as a slowing or stopping of the progression of the tumor or cancer or metastatic cancer of the patient.

As used herein, the term "inhibition", as it relates to cancer and/or cancer cell proliferation, refers to the inhibition of the growth, division, maturation or viability of cancer cells, and/or causing the death of cancer cells, individually or in aggregate with other cancer cells, by cytotoxicity, nutrient depletion, or the induction of apoptosis.

As used herein, "delaying" the onset or "delaying" reoccurrence of a disease or disorder, or one or more symptoms thereof, means to defer, hinder, slow, retard, stabilize and/or postpone development of the disease, disorder, or symptom thereof. This delay can be of varying lengths of time, depending on the history of the disease and/or subject being treated. As it is evident to one skilled in the art, a sufficient or significant delay can, in effect, encompass prevention, in that the subject does not develop the disease, disorder, or symptom thereof. For example, a method that "delays" development of cancer or reoccurrence of the cancer is a method that reduces the probability of disease development in a given time frame and/or reduces extent of the disease in a given time frame, when compared to not using the described method. Such comparisons may be based on clinical studies, using a statistically significant number of subjects.

In some embodiments, treatment may be administered after one or more symptoms have developed. In other embodiments, treatment may be administered in the absence of symptoms. For example, a treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Also provided herein are methods of enhancing, improving and/or increasing the response to an anticancer therapy, in particular a bispecific antibody construct comprising at least one first binding domain capable of specifically binding CD16A and at least one second binding domain capable of specifically binding EGFR, and a taxane in a subject suffering from a tumor.

As used herein, the term "bispecific antibody construct" refers to an antibody construct which is bispecific, i.e., it comprises at least a first binding domain and a second binding domain, wherein the first binding domain binds to one antigen or target (here: NK cell receptor CD16A), and the second binding domain binds to another antigen or target (here: the target cell surface antigen EGFR).

Examples of bispecific antibody constructs are provided, for example, in WO 2006/125668, WO 2017/064221, WO 2019/175368, WO 2019/198051, WO 2020/043670, WO 2021/130383, and Ellwanger et a. (MAbs. 2019 Jul;11(5):899-918).

A bispecific antibody or an antigen binding fragment thereof may comprise a first binding domain capable of specifically binding CD16A and a second binding domain capable of specifically binding EGFR. The multispecific antibody construct disclosed herein may comprise a CD16A binding domain and an EGFR binding domain.

Given that the antibody constructs as defined in the context of the invention are (at least) bispecific, they do not occur naturally and they are markedly different from naturally occurring products. A "bispecific" antibody construct or immunoglobulin is hence an artificial hybrid antibody or immunoglobulin having at least two distinct binding sides with different specificities. Bispecific antibody constructs can be produced by a variety of methods including fusion of hybridomas or linking of Fab' fragments. See, e.g., Songsivilai & Lachmann, Clin. Exp. lmmunol. 79:315- 321 (1990).

As used herein, "CD16A" refers to the activating receptor CD16A, also known as FcyRIIIA, expressed on the cell surface of NK cells. CD16A is an activating receptor triggering the cytotoxic activity of NK cells. The affinity of antibodies for CD16A directly correlates with their ability to trigger NK cell activation, thus higher affinity towards CD16A reduces the antibody dose required for activation. The antigen-binding site of the antigen-binding protein binds to CD16A, but not to CD16B. For example, an antigen-binding site comprising heavy (VH) and light (VL) chain variable domains binding to CD16A, but not binding to CD16B, may be provided by an antigen binding site which specifically binds to an epitope of CD16A which comprises amino acid residues of the C-terminal sequence SFFPPGYQ (SEQ ID NO: 70) and/or residues G130 and/or Y141 of CD16A (SEA ID NO: 71) which are not present in CD16B. In some embodiments, the antigen-binding site for CD16A does not bind to CD16B and binds to the known CD16A allotypes F158 and V158 with similar affinity. Two allelic single nucleotide polymorphisms have been identified in human CD16A altering the amino acid in position 158, which is important for interaction with the hinge region of IgG. The allelic frequencies of the homozygous 158 F/F and the heterozygous 158 V/F alleles are similar within the Caucasian population, ranging between 35 and 52% or 38 and 50%, respectively, whereas the homozygous 158 V/V allele is only found in 10-15% (Lopez-Escamez JA et al.; BMC Med Genet 2011; 12: 2). Activation of NK-cells by this anti-CD16A domain in all patients due to the similar affinity is therefore advantageous. Further CD16A antigen-binding sites comprising heavy and light variable chain domains that bind to CD16A, but not to CD16B are described in WO 2006/125668.

The term "EGFR" refers to the epidermal growth factor receptor (EGFR; ErbB-1; HER1) in humans, including all isoforms or variants described with activation, mutations and implicated in pathophysiological processes. A binding domain specific for EGFR may recognize an epitope in the extracellular domain of the EGFR. A binding domain specific for EGFR may specifically bind to human and cynomolgus EGFR, i.e. the binding domain may be cross-reactive to human and cynomolgus EGFR. The epidermal growth factor receptor (EGFR) is a member of the HER family of receptor tyrosine kinases and consists of four members: EGFR (ErbB1/HER1), HER2/neu (ErbB2), HER3 (ErbB3) and HER4 (ErbB4). Stimulation of the receptor through ligand binding (e.g. EGF, TGFa, HB-EGF, neuregulins, betacellulin, amphiregulin) activates the intrinsic receptor tyrosine kinase in the intracellular domain through tyrosine phosphorylation and promotes receptor homo- or heterodimerization with HER family members. These intracellular phospho-tyrosines serve as docking sites for various adaptor proteins or enzymes including SHC, GRB2, PLCg and PI(3)K/Akt, which simultaneously initiate many signaling cascades that influence cell proliferation, angiogenesis, apoptosis resistance, invasion and metastasis. EGFR can be expressed by a number of different tumor types including lung (particularly non-small cell cancer NSCLC), colorectal (particularly CRC), liver (particularly hepatocellular carcinoma HCC), brain (particularly glioblastoma GBM), kidney/renal (particularly ccRCC), ovarian, breast (particularly TNBC), squamous cell carcinoma (particularly squamous cervical cancer), bladder, head and neck (particularly squamous cell carcinoma of head and neck SCCHN), gastric cancer, esophagus, sarcoma, mesothelioma, and adenocarcinoma.

The term "epitope" refers to a side on an antigen to which a binding domain, such as an antibody or immunoglobulin, or a derivative, fragment or variant of an antibody or an immunoglobulin, specifically binds. An "epitope" is antigenic and thus the term epitope is sometimes also referred to herein as "antigenic structure" or "antigenic determinant". Thus, the binding domain is an "antigen interaction site". Said binding/interaction is also understood to define a "specific recognition". "Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence. A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically, a conformational epitope comprises an increased number of amino acids relative to a linear epitope.

As used herein, "CD16B" refers to receptor CD16B, also known as FcyRIIIB, expressed on neutrophils and eosinophils. The receptor is glycosylphosphatidyl inositol (GPI) anchored and is understood to not trigger any kind of cytotoxic activity of CD16B positives immune cells.

In some embodiments, the first binding domain of the multispecific antibody construct used in the context of the means and methods of the present invention binds to an epitope on CD16A which is C-terminal to the physiological Fcy receptor binding domain, said epitope preferably comprising Y158 of SEQ ID NO: 1.

The term "binding domain" in connection with the present invention characterizes a domain which is capable of specifically binding to / interacting with / recognizing a given target epitope or a given target site on the target molecules (antigens), e.g. the NK cell receptor antigen CD16A on the surface of the NK cell, and a target cell surface antigen, i.e. EGFR on the tumor cell, respectively. Thus, in some embodiments, the multispecific antibody construct comprises at least a first binding domain capable of specifically binding CD16A on the surface of an immune effector and at least a second binding domain capable of specifically binding EGFR on the surface of a tumor cell.

The structure and/or function of the first binding domain (recognizing CD16A), and preferably also the structure and/or function of the second binding domain (recognizing the target cell surface antigen EGFR), is/are based on the structure and/or function of an antibody, e.g. of a full-length or whole immunoglobulin molecule and/or is/are drawn from the variable heavy chain (VH) and/or variable light chain (VL) domains of an antibody or fragment thereof. Preferably the first binding domain is characterized by the presence of three light chain CDRs (i.e. CDR1, CDR2 and CDR3 of the VL region) and/or three heavy chain CDRs (i.e. CDRl, CDR2 and CDR3 of the VH region). The second binding domain preferably also comprises the minimum structural requirements of an antibody which allow for the target binding. More preferably, the second binding domain comprises at least three light chain CDRs (i.e. CDR1, CDR2 and CDR3 of the VL region) and/or three heavy chain CDRs (i.e. CDRl, CDR2 and CDR3 of the VH region). In some cases, the first and/or second binding domain is produced by or obtainable by phage-display or library screening methods rather than by grafting CDR sequences from a pre-existing (monoclonal) antibody into a scaffold.

The term "variable" refers to the portions of the antibody or immunoglobulin domains that exhibit variability in their sequence and that are involved in determining the specificity and binding affinity of a particular antibody (i.e., the "variable domain(s)"). The pairing of a variable heavy chain (VH) and a variable light chain (VL) together forms a single antigen- binding side.

Variability is not evenly distributed throughout the variable domains of antibodies; it is concentrated in sub-domains of each of the heavy and light chain variable regions. These sub-domains are called "hypervariable regions" or "complementarity determining regions" (CDRs). The more conserved (i.e., non-hypervariable) portions of the variable domains are called the "framework" regions (FRM or FR) and provide a scaffold for the six CDRs in three-dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding side (see Kabat et al., loc. cit.).

The terms "CDR", and its plural "CDRs", refer to the complementarity determining region of which three make up the binding character of a light chain variable region (CDR-L1, CDR-L2 and CDR-L3) and three make up the binding character of a heavy chain variable region (CDR-H1, CDR-H2 and CDR-H3). CDRs contain most of the residues responsible for specific interactions of the antibody with the antigen and hence contribute to the functional activity of an antibody molecule: they are the main determinants of antigen specificity. The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. *In vitro* selection schemes in which CDR3 alone is varied can be used to vary the binding properties of an antibody or determine which residues contribute to the binding of an antigen. Hence, CDR3 is typically the greatest source of molecular diversity within the antibody-binding side. H3, for example, can be as short as two amino acid residues or greater than 26 amino acids.

The exact definitional CDR boundaries and lengths are subject to different classification and numbering systems. CDRs may therefore be referred to by IMGT, Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example the IMGT method as described in Lefranc, M.-P., The Immunologist, 7, 132-136 (1999), Kabat (an approach based on cross-species sequence variability), Chothia (an approach based on crystallographic studies of antigen-antibody complexes), and/or MacCallum (Kabat et al., loc. cit; Chothia et al., J. Mol. Biol, 1987, 196: 901 -917; and MacCallum et al., J. Mol. Biol, 1996, 262: 732). Still another standard for characterizing the antigen binding side is the AbM definition used by Oxford Molecular's AbM antibody modeling software. See, e.g., Protein Sequence and Structure Analysis of Antibody Variable Domains. In: Antibody Engineering Lab Manual (Ed.: Duebel, S. and Kontermann, R., Springer-Verlag, Heidelberg). To the extent that two residue identification techniques define regions of overlapping, but not identical regions, they can be combined to define a hybrid CDR. However, the numbering in accordance with the so-called IMGT system or the Kabat system is preferred.

As used herein, the term "target cell surface antigen" refers to an antigenic structure expressed by a cell and which is present at the cell surface such that it is accessible for an antibody construct as described herein. In the context of the present invention, "target cell surface antigen" to which the bispecific antibody constructs described herein binds to is EGFR.

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

Preferably the binding domain which binds to the NK cell receptor antigen (CD16A) and/or the binding domain which binds to the target cell surface antigen EGFR is/are human, humanized or murine derived chimeric binding domains. Antibodies and antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. In order to avoid the use of rodent derived antibodies or antibody constructs, human or fully human antibodies/ antibody constructs can be generated through the introduction of human antibody function into a rodent so that the rodent produces fully human antibodies.

In some embodiments, the at least one first binding domain described herein comprises means for specifically binding CD16A and the at least one second binding domain comprises means for specifically binding EGFR. In some embodiments the bispecific antibody construct comprises means for specifically binding CD16A and EGFR.

The term "specifically binding", as used herein means that the binding domain preferentially binds or recognizes the target even when the binding partner is present in a mixture of other molecules or other structures. The binding may be mediated by covalent or non-covalent interactions or a combination of both. In preferred examples, "simultaneous binding to a target cell and an immune effector cell" comprises the physical interaction between the binding domains and their targets on the cells, but preferably also includes the induction of an action mediated by the simultaneous binding of the two cells. Such an action may be an immune effector function of the immune effector cell, such as a cytotoxic effect. "Means" for specifically binding CD16A and EGFR are structures comprised by a binding domain that help to interact with the target molecule. Said structures are preferably one or more polypeptides described elsewhere herein.

The term "antibody construct" refers to a molecule in which the structure and/or function is/are based on the structure and/or function of an antibody, e.g., of a full-length or whole immunoglobulin molecule and/or is/are drawn from the variable heavy chain (VH) and/or variable light chain (VL) domains of an antibody or fragment thereof. An antibody construct is hence capable of binding to its specific target or antigen. Furthermore, the binding region of an antibody construct comprises the minimum structural requirements of an antibody which allow for the target binding. For the first binding domain to CD16A, this minimum requirement is defined by the presence of a VL region comprising the three light chain CDRs (i.e. CDR1, CDR2 and CDR3 of the VL region) and the presence of a VH region comprising the three heavy chain CDRs (i.e. CDR1, CDR2 and CDR3 of the VH region). For the second binding domain to a tumor antigen, this minimum requirement may, e.g., be defined by the presence of at least the three light chain CDRs (i.e. CDR1, CDR2 and CDR3 of the VL region) and/or the three heavy chain CDRs (i.e. CDR1, CDR2 and CDR3 of the VH region), preferably of all six CDRs. An alternative approach to define the minimal structure requirements of an antibody is the definition of the epitope of the antibody within the structure of the specific target, respectively, the protein domain of the target protein composing the epitope region (epitope cluster) or by reference to a specific antibody competing with the epitope of the defined antibody. The antibodies on which the constructs defined in the context of the invention are based include for example monoclonal, recombinant, chimeric, deimmunized, humanized and human antibodies.

The first binding domain of an antibody construct comprises the designated groups of CDRs. Those CDRs can be comprised in the framework of an antibody light chain variable region (VL) and an antibody heavy chain variable region (VH). The second binding domain comprises defined groups of CDRs. Preferably, those CDRs are comprised in the framework of an antibody light chain variable region (VL) and an antibody heavy chain variable region (VH); however, it does not have to comprise both. Fd fragments, for example, have two VH regions and often retain some antigen-binding function of the intact antigen-binding region.

Examples for the format of antibody fragments, antibody variants or binding domains include (1) a Fab fragment, a monovalent fragment having the VL, VH, CL and CH1 domains; (2) a F(ab')₂fragment, a bivalent fragment having two Fab fragments linked by a disulfide bridge at the hinge domain; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv), the latter being preferred (for example, derived from an scFv-library). Examples for antibody constructs are e.g. described in WO00/006605, WO2005/040220, WO2008/119567, WO2010/037838, WO2013/026837, WO2013/026833, US2014/0308285, US2014/0302037, WO2014/144722, WO2014/151910, and WO2015/048272.

An antibody construct that can be used in methods of the disclosure can comprise a fragment of a full-length antibody, such as VH, VHH, VL, (s)dAb, Fv, Fd, Fab, Fab', F(ab')₂ or "r IgG" ("half antibody"). Antibody constructs can also comprise modified fragments of antibodies, also called antibody variants, such as scFv, di-scFv or bi(s)-scFv, scFv-Fc, scFv-zipper, scFab, Fab₂, Fabs, diabodies, single chain diabodies, tandem diabodies (Tandab's), tandem di-scFv, tandem tri-scFv, "multibodies" such as triabodies or tetrabodies, and single domain antibodies such as nanobodies or single variable domain antibodies comprising merely one variable domain, which might be VHH, VH or VL, that specifically bind an antigen or epitope independently of other V regions or domains.

As used herein, the terms "single-chain Fv," "single-chain antibodies" or "scFv" refer to single polypeptide chain antibody fragments that comprise the variable regions from both the heavy and light chains, but lack the constant regions. Generally, a single-chain antibody further comprises a polypeptide linker between the VH and VL domains which enables it to form the desired structure which would allow for antigen binding. Exemplary linkers for this purpose include glycine serine linkers, which preferably comprises from about 15 to about 30 amino acids. Preferred glycine serine linkers may have one or more repeats of GGS, GGGS (SEQ ID NO: 72), or GGGGS (SEQ ID NO: 5). Such linker preferably comprises 5, 6, 7, 8, 9 and/or 10 repeats of GGS, preferably (GGS)₆ (SEQ ID NO:2, which are preferably used for scFvs having the arrangement VH-VL), or preferably (GGS)₇ (SEQ ID NO: 3, which are preferably used for scFvs having the arrangement VL-VH). Single chain antibodies are discussed in detail by Plueckthun in The Pharmacology of Monoclonal Antibodies, vol. 1 13, Rosenburg and Moore eds. Springer-Verlag, New York, pp. 269-315 (1994). Various methods of generating single chain antibodies are known, including those described in U.S. Pat. Nos. 4,694,778 and 5,260,203; International Patent Application Publication No. WO 88/01649; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038- 1041. In specific embodiments, single-chain antibodies can also be human, and/or humanized and/or synthetic. The term "bi-scFv" or "ta-scFv" (tandem scFv) as used herein refers to two scFv that are fused together. Such a bi-scFv or ta-scFv may comprise a linker between the two scFv moieties. Generally, the arrangement of the VH and VL domains on the polypeptide chain within each of the scFv may be in any order. This means that the "bi-scFv" of "ta-scFv" can be arranged in the order VH(1)-VL(1)-VH(2)-VL(2), VL(1)-VH(1)-VH(2)-VL(2), VH(1)-VL(1)-VL(2)-VH(2), or VL(1)-VH(1)-VL(2)-VH(2), where (1) and (2) stand for the first and second scFv, respectively. The term "double Fab" as used herein refers to two Fab fragments that are fused together, which are preferably staggered. Here, a first chain of a first Fab is N-terminally fused to a first chain of a second Fab, or a second chain of a first Fab is N-terminally fused to a second chain of a second Fab, or both, the first chain of a first Fab and the second chain of a first Fab are fused to first and second chains of a second Fab, respectively. A linker may be present between the fused chains of the first and second Fab. The first and second chains of the first and second Fab can be individually selected from a light chain-derived chain of a Fab (VL-CL), a heavy chain derived chain of a Fab (VH-CH1), as long as each Fab contains a VH, a VL, a CH1, and a CL. As an illustrative example, the light chain-derived chain of the first Fab can be fused to the light chain derived-chain of the second Fab. As another illustrative example, the heavy chain-derived chain of the first Fab can be fused to the heavy chain derived-chain of the second Fab. As a further illustrative example, the heavy chain-derived chain of the first Fab can be fused to the light chain derived-chain of the second Fab. In some double Fabs, both chains of the two Fabs are fused together. For example, the light chain-derived chain of the first Fab can be fused to the light chain derived-chain of the second Fab while the heavy chain-derived chain of the first Fab can be fused to the heavy chain derived-chain of the second Fab. Alternatively, the light chain-derived chain of the first Fab can be fused to the heavy chain derived-chain of the second Fab while the heavy chain-derived chain of the first Fab can be fused to the light chain derived-chain of the second Fab.

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 9); a heavy chain complementarity determining region 2 (CDR-H2) having the sequence INPSGGST (SEQ ID NO: 10); and a heavy chain complementarity determining region 3 (CDR-H3) having the sequence ARGSAYYYDFADY (SEQ ID NO: 11), and a light chain variable domain (VL_CD16a) comprising a light chain complementarity determining region 1 (CDR-L1) having the sequence NIGSKN (SEQ ID NO: 12), a light chain complementarity determining region 2 (CDR-L2) having the sequence QDN; and a light chain complementarity determining region 3 (CDR-L3) having the sequence QVWDNYSVL (SEQ ID NO: 13).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 17); a heavy chain complementarity determining region 2 (CDR-H2) having the sequence IEPMYGST (SEQ ID NO: 18); and a heavy chain complementarity determining region 3 (CDR-H3) having the sequence ARGSAYYYDFADY (SEQ ID NO: 19), and a light chain variable domain (VL_CD16a) comprising a light chain complementarity determining region 1 (CDR-L1) having the sequence NIGSKN (SEQ ID NO: 20), a light chain complementarity determining region 2 (CDR-L2) having the sequence QDN; and a light chain complementarity determining region 3 (CDR-L3) having the sequence QVWDNYSVL (SEQ ID NO: 21).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTNYY (SEQ ID NO: 25); a heavy chain complementarity determining region 2 (CDR-H2) having the sequence INPSGGVT (SEQ ID NO: 26); and a heavy chain complementarity determining region 3 (CDR-H3) having the sequence ARGSAYYYDFADY (SEQ ID NO: 27), and a light chain variable domain (VL_CD16a) comprising a light chain complementarity determining region 1 (CDR-L1) having the sequence NIGSKS (SEQ ID NO: 28), a light chain complementarity determining region 2 (CDR-L2) having the sequence QDK; and a light chain complementarity determining region 3 (CDR-L3) having the sequence QVWDDYIVL (SEQ ID NO: 29).

In some embodiments, the binding domain specifically binding CD16A of the bispecific antibody construct comprises a heavy chain variable domain (VH_CD16a) comprising a heavy chain complementarity determining region 1 (CDR-H1) having the sequence SYYMH (SEQ ID NO: 32); a heavy chain complementarity determining region 2 (CDR-H2) having the sequence AIEPRYGSTSYAQKFQG (SEQ ID NO: 33); and a heavy chain complementarity determining region 3 (CDR-H3) having the sequence GSAYYYDFADY (SEQ ID NO: 34), and a light chain variable domain (VL_CD16a) comprising a light chain complementarity determining region 1 (CDR-L1) having the sequence GGHNIGSKNVH (SEQ ID NO: 35), a light chain complementarity determining region 2 (CDR-L2) having the sequence QDNKRPS (SEQ ID NO: 36); and a light chain complementarity determining region 3 (CDR-L3) having the sequence QVWDNYNVL (SEQ ID NO: 37).

In some embodiments, the binding domain specifically binding CD16A of the bispecific antibody construct comprises a heavy chain variable domain (VH_CD16a) comprising a heavy chain complementarity determining region 1 (CDR-H1) having the sequence NYYMQ (SEQ ID NO: 38); a heavy chain complementarity determining region 2 (CDR-H2) having the sequence IINPSGGVTSYAQKFQG (SEQ ID NO: 39); and a heavy chain complementarity determining region 3 (CDR-H3) having the sequence GSAYYYDFADY (SEQ ID NO: 40), and a light chain variable domain (VL_CD16a) comprising a light chain complementarity determining region 1 (CDR-L1) having the sequence GGNNIGSKSVH (SEQ ID NO: 41), a light chain complementarity determining region 2 (CDR-L2) having the sequence QDKKRPS (SEQ ID NO: 42); and a light chain complementarity determining region 3 (CDR-L3) having the sequence QVWDDYIVL (SEQ ID NO: 43).

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 14; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 15.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 22; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 23.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 30; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 31.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 44; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 45.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 46; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 47.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 48; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 49.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 50; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 51.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 52; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 53.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 54; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 55.

In some embodiments, the binding domain specifically binding CD16A comprises a heavy chain variable (VH) region comprising or consisting of SEQ ID NO: 14; and a light chain variable (VL) region comprising or consisting of SEQ ID NO: 15.

In some embodiments, the binding domain specifically binding CD16A comprises a heavy chain variable (VH) region comprising or consisting of SEQ ID NO: 22; and a light chain variable (VL) region comprising or consisting of SEQ ID NO: 23.

In some embodiments, the binding domain specifically binding CD16A comprises a heavy chain variable (VH) region comprising or consisting of SEQ ID NO: 30; and a light chain variable (VL) region comprising or consisting of SEQ ID NO: 31.

In some embodiments, the binding domain specifically binding CD16A comprises a heavy chain variable (VH) region comprising or consisting of SEQ ID NO: 44; and a light chain variable (VL) region comprising or consisting of SEQ ID NO: 45.

In some embodiments, the binding domain specifically binding CD16A comprises a heavy chain variable (VH) region comprising or consisting of SEQ ID NO: 46; and a light chain variable (VL) region comprising or consisting of SEQ ID NO: 47.

In some embodiments, the binding domain specifically binding CD16A comprises a heavy chain variable (VH) region comprising or consisting of SEQ ID NO: 48; and a light chain variable (VL) region comprising or consisting of SEQ ID NO: 49.

In some embodiments, the binding domain specifically binding CD16A comprises a heavy chain variable (VH) region comprising or consisting of SEQ ID NO: 50; and a light chain variable (VL) region comprising or consisting of SEQ ID NO: 51.

In some embodiments, the binding domain specifically binding CD16A comprises a heavy chain variable (VH) region comprising or consisting of SEQ ID NO: 52; and a light chain variable (VL) region comprising or consisting of SEQ ID NO: 53.

In some embodiments, the binding domain specifically binding CD16A comprises a heavy chain variable (VH) region comprising or consisting of SEQ ID NO: 54; and a light chain variable (VL) region comprising or consisting of SEQ ID NO: 55.

In some embodiments, the first binding domain is a variable domain (Fv). In some embodiments, the first binding domain is a single chain Fv (scFv). In some embodiments, the first binding domain is a Fab. In some embodiments, the first binding domain is a single chain diabody (scDb). In some embodiments, the first binding domain is a diabody (Db). In some embodiments, the first binding domain is a double Fab. Preferably, the first binding domain is a Fab.

In some embodiments, the binding domain specifically binding EGFR of the bispecific antibody construct comprises a heavy chain variable domain (VH EGFR) comprising a heavy chain complementarity determining region 1 (CDR-H1) having the sequence GGSVSSGSYY (SEQ ID NO: 56); a heavy chain complementarity determining region 2 (CDR-H2) having the sequence IYYSGST (SEQ ID NO: 57); and a heavy chain complementarity determining region 3 (CDR-H3) having the sequence ARNPISIPAFDI (SEQ ID NO: 58), and a light chain variable domain (VL_ EGFR) comprising a light chain complementarity determining region 1 (CDR-L1) having the sequence NIGSKS (SEQ ID NO: 59), a light chain complementarity determining region 2 (CDR-L2) having the sequence YDS; and a light chain complementarity determining region 3 (CDR-L3) having the sequence QVWDTSSDHVL (SEQ ID NO: 60).

In some embodiments, the binding domain specifically binding EGFR comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 61; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 62.

In some embodiments, the binding domain specifically binding EGFR comprises a heavy chain variable (VH) region comprising or consisting of SEQ ID NO: 61; and a light chain variable (VL) region comprising or consisting of SEQ ID NO: 62.

In some embodiments, the second binding domain is a variable domain (Fv). In some embodiments, the second binding domain is a single chain Fv (scFv). In some embodiments, the second binding domain is a Fab. In some embodiments, the second binding domain is a single chain diabody (scDb). In some embodiments, the second binding domain is a diabody (Db). In some embodiments, the second binding domain is a double Fab. Preferably, the second binding domain is a scFv.

In some embodiments, the multispecific antibody construct binds to a target cell and an immune effector cell simultaneously, as described elsewhere herein.

In some embodiments, the multispecific antibody construct comprises a third domain comprising a half-life extension domain. In some embodiments, the half-life extension domain comprises a CH2 domain, wherein the Fcγ receptor binding domain is optionally silenced. In some embodiments, the half-life extension domain comprises a CH3 domain.

As used herein the term "half-life extensions domain" relates to a moiety that prolongs serum half-life of the antibody construct. The half-life extension domain may comprise a portion of an antibody, such as an Fc part of an immunoglobulin, a hinge domain, a CH2 domain, a CH3 domain, and/or a CH4 domain. Although less preferred, a half-life extension domain can also comprise elements that are not comprised in an antibody, such as an albumin binding peptide, an albumin binding protein, or transferrin to name only a few. A half-life extension domain preferably does not have an immune-modulatory function. If a half-life extension domain comprises a hinge, CH2 and/or CH3 domain, the half-life extension domain preferably does not essentially bind to an Fc receptor. This can e.g. be achieved through "silencing" of the Fcy receptor binding domain.

As used herein, "silencing" of the Fc or Fcy receptor binding domain refers to any modification that reduces binding of a CH2 domain to an Fc receptor, in particular an Fcy receptor. Such modification can be done by replacement and/or deletion of one or more amino acids that are involved in Fc(y) receptor-binding. Such mutations are well known in the art and have e.g. been described by Saunders (2019, Front. Immunol. 10:1296). For example, a mutation can be located at any one of the positions 233, 234, 235, 236, 237, 239, 263, 265, 267, 273, 297, 329, and 331. Examples for such mutations are: deletion of Glu 233 -> Pro, Glu 233, Leu 234 -> Phe, Leu 234 - > Ala, Leu 234 -> Gly, Leu 234 -> Glu, Leu 234 -> Val, deletion of Leu 234, Leu 235 -> Glu, Leu 235 -> Ala, Leu 235 -> Arg, Leu 235 -> Phe, deletion of Leu 235, deletion of Gly 236, Gly 237 - > Ala, Ser 239 -> Lys, Val 263 -> Leu, Asp 265 -> Ala, Ser 267 -> Lys, Val 273 -> Glu, Asn 297 -> Gly, Asn 297 -> Ala, Lys 332 -> Ala, Pro 329 -> Gly, Pro 331 -> Ser and combinations thereof. Preferably, such a modification comprises one or both of Leu 234 -> Ala and Leu 235 -> Ala (also known as "LALA" mutation). Preferably, such a modification further comprises a Pro 329 -> Gly mutation, also known as "LALA-PG" mutation (Leu 234 -> Ala, Leu 235 -> Ala, and Pro 329 -> Gly). Preferably, such a modification comprises 1, 2, or 3 of the mutations Leu 234 -> Phe, Leu 235 -> Glu, and Asp 265 -> Ala, more preferably all three of these mutations. The combination Leu 234 -> Phe, Leu 235 -> Glu, and Asp 265 -> Ala, which is a preferred modification in the context of the present invention, is also known as "FEA" mutation. Preferably, such a modification further comprises Asn 297 -> Gly. Such a preferred modification comprises the mutations Leu 234 -> Phe, Leu 235 -> Glu, Asp 265 -> Ala, and Asn 297 -> Gly.

In some embodiments, the half-life extension domain may comprise two CH3 domains. The half-life extension domain may comprise a hinge domain. The half-life extension domain may comprise two hinge domains. The half-life extension domain may comprise a CH2 domain and a CH3 domain. In such a case, the CH2 domain and CH3 domain are preferably fused to each other, preferably in the (amino to carboxyl) order CH2 domain - CH3 domain. The half-life extension domain may comprise a hinge domain and a CH2 domain. In such a case, the hinge domain and the CH2 domain are preferably fused to each other, preferably in the (amino to carboxyl) order hinge domain - CH2 domain. The half-life extension domain may comprise a hinge domain, a CH2 domain, and a CH3 domain. In such a case, the hinge domain, the CH2 domain, and CH3 domain are preferably fused to each other, preferably in the (amino to carboxyl) order hinge domain - CH2 domain - CH3 domain. The half-life extending domain may comprise two hinge domain - CH2 domain elements, two CH2 domain - CH3 domain elements, or two hinge domain - CH2 domain - CH3 domain elements. In such a case the two fusions may be located on two different polypeptide strands. Alternatively, the fusions can be located on the same polypeptide strand. An illustrative example for two hinge domain - CH2 domain - CH3 domain elements that are located on the same polypeptide strand is the "single chain Fc" or "scFc" format. Here, both hinge-CH2-CH3 subunits are fused together via a linker that allows assembly of a Fc domain. A preferred linker for this purpose is a glycine serine linker, which preferably comprises from about 20 to about 40 amino acids. Preferred glycine serine linkers may have one or more repeats of GGS, GGGS (SEQ ID NO: 72), or GGGGS (SEQ ID NO: 5). Such linker preferably comprises 4-8 repeats (e.g. 4, 5, 6, 7, or 8 repeats) of GGGGS. Such a linker is preferably (GGGGS)₆, (SEQ ID NO 8). Further scFc constant domains are known in the art and *inter alia* described in WO 2017/134140.

Generally, the bispecific antibody constructs used in accordance with the present invention can be monovalent, bivalent, trivalent, or have an even higher valency for any one of the first target (CD16A) and the second target (EGFR). The antibody constructs of the disclosure may thus comprise one, two, three, or even more of any one of the first binding domain and the second binding domain. It is preferred for the antibody construct of the invention that it is at least monovalent for the first target (CD16A) and at least monovalent for the second target (EGFR). It is also preferred for the antibody construct of the invention that it is at least monovalent for the first target (CD16A) and bivalent for the second target (EGFR). It is further preferred for the antibody construct of the invention that it is at least bivalent for the first target (CD16A) and at least bivalent for the second target (EGFR). It also preferred for the antibody construct of the invention that it is at least bivalent for the first target (CD16A) and at least trivalent for the second target (EGFR). It also preferred for the antibody construct of the invention that it is at least bivalent for the first target (CD16A) and at least monovalent for the second target (EGFR). Most preferred, the antibody construct of the invention is bivalent for the first target (CD16A) and bivalent for the second target (EGFR).

In some embodiments, the bispecific antibody construct in accordance with the present invention comprises at least one first binding domain and at least one second binding domain. In some embodiments, the multispecific antibody construct comprises at least one first binding domain and at least two second binding domain. It is further preferred for the multispecific antibody construct that it comprises at least two first binding domains and at least two second binding domains. It is further preferred for the multispecific antibody construct that it comprises at least two first binding domains and at least three second binding domains. It is further preferred for the multispecific antibody construct that it comprises at least two first binding domains and at least one second binding domain. Most preferred, the multispecific antibody construct comprises two first binding domains and two second binding domains.

In some embodiments, the first binding domain or the second binding domain of the bispecific antibody construct may be fused to a constant domain of an antibody via a linker. Such a linker is preferably a short linker, which preferably has a length of about 10 nm or less, preferably about 9 nm or less, preferably about 8 nm or less, preferably about 7 nm or less, preferably about 6 nm or less, preferably about 5 nm or less, preferably about 4 nm or less, or even less. The length of the linker is preferably determined as described by Rossmalen et al Biochemistry 2017, 56, 6565-6574, which also describes suitable linkers that are well known to the skilled person. An example for a suitable linker is a glycine serine linker or a serine linker, which preferably comprise no more than about 75 amino acids, preferably not more than about 50 amino acids. In illustrative example, a suitable linker comprises one or more (e.g. 1, 2, 3, 4, 5, 6, 7, or 8) GGGGS sequences (SEQ ID NO: 5), such as (GGGGS)₄ (SEQ ID NO: 7), (GGGGS)₆ (SEQ ID NO: 8), or preferably (GGGGS)₂ (SEQ ID NO: 6). Other illustrative examples for linkers are shown in SEQ ID NOs: 1-8 and 72.

In some embodiments, the multispecific antibody construct may also comprise additional domains, which are e.g. helpful in the isolation of the molecule or relate to an adapted pharmacokinetic profile of the molecule. Domains helpful for the isolation of an antibody construct may be selected from peptide motives or secondarily introduced moieties, which can be captured in an isolation method, e.g. an isolation column. Non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. StrepII-tag) and His-tag. All herein disclosed antibody constructs characterized by the identified CDRs may comprise a His-tag domain, which is generally known as a repeat of consecutive His residues in the amino acid sequence of a molecule, preferably of five, and more preferably of six His residues (hexa-histidine). The His-tag may be located e.g. at the N- or C-terminus of the antibody construct, preferably it is located at the C-terminus. Most preferably, a hexa-histidine tag is linked via peptide bond to the C-terminus of the antibody construct according to the invention. Additionally, a conjugate system of PLGA-PEG-PLGA may be combined with a poly-histidine tag for sustained release application and improved pharmacokinetic profile. In some embodiments the histidine tag is a hexa-histidine tag (SEQ ID NO: 73).

Amino acid sequence modifications of the antibody constructs described herein are also contemplated, as long as the minimal structural limitations of the first binding domain of the antibody construct of the present invention are maintained. For example, it may be desirable to improve the binding affinity and/or other biological properties of the antibody construct. Amino acid sequence variants of the antibody constructs are prepared by introducing appropriate nucleotide changes into the antibody constructs nucleic acid, or by peptide synthesis. All of the below described amino acid sequence modifications should result in an antibody construct which still retains the desired biological activity (i.e. at least binding to CD16A and EGFR) of the unmodified parental molecule.

Amino acid modifications include, for example, deletions from, and/or insertions into, and/or substitutions of, residues within the amino acid sequences of the antibody constructs. Any combination of deletion, insertion, and substitution is made to arrive at the final construct, provided that the final construct possesses the desired characteristics. The amino acid changes also may alter post-translational processes of the antibody constructs, such as changing the number or position of glycosylation sites.

Generally, if amino acids are substituted in one or more or all of the CDRs of the heavy and/or light chain, it is preferred that the then-obtained "substituted" sequence is at least 60% or at least 65%, more preferably at least 70% or at least 75%, even more preferably at least 80% or at least 85%, and particularly preferably at least 90% or at least 95% identical to the "original" CDR sequence. This means that it is dependent of the length of the CDR to which degree it is identical to the "substituted" sequence. For example, a CDR having 5 amino acids is preferably at least 80% identical to its substituted sequence in order to have at least one amino acid substituted. Accordingly, the CDRs of the antibody construct may have different degrees of identity to their substituted sequences, e.g., CDRL1 may have at least 80%, while CDRL3 may have at least 90%. Preferred substitutions (or replacements) are conservative substitutions. However, any substitution (including non-conservative substitution) is envisaged as long as the antibody construct retains its capability to bind to CD16A via the first binding domain, and/or to the target cell surface antigen via the second binding domain and/or the CDRs of the second binding domain have an identity to the then substituted sequence (at least 60% or at least 65%, more preferably at least 70% or at least 75%, even more preferably at least 80% or at least 85%, and particularly preferably at least 90% or at least 95% identical to the "original" CDR sequence). Conservative substitutions are shown in **Table 1** under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino acid substitutions**

| **Original** | **Exemplary Substitutions** | **Preferred Substitutions** |
|---|---|---|
| Ala (A) | val, leu, i!e | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | a rg |
| Ile(I) | leu, val, met, ala, phe | leu |
| Leu (L) | norleucine, ile, va!, met, ala | lie |
| Lys (K) | arg, gln, asn | a rg |
| Met (M) | leu, phe, i!e | leu |
| Phe (F) | leu, val, ile, a!a, tyr | tyr |
| Pro (P) | ala | ala |
| ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Non-conservative substitutions will entail exchanging a member of one of these classes for another class. Any cysteine residue not involved in maintaining the proper conformation of the antibody construct may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

In preferred embodiments, the second binding domain of the multispecific antibody construct is preferably a scFv fragment that is fused to a C terminus of a Fc domain, preferably via the VH domain of the scFv. Accordingly, the arrangement of the polypeptide chain (from N to C) is preferably ... -CH2-CH3-VH-VL, optionally with a linker between the Fc and the scFv. The first binding domain can be located at any suitable position of the antibody construct. Where the antibody construct comprises a Fc region, the first binding domain can be located N terminal of the Fc region, either directly or linked via at least a part of a hinge domain. Other linkers disclosed herein can also be used to link the first binding domain to the Fc domain. A hinge domain is however preferred for this purpose. The first binding domain can be any suitable structure disclosed herein, while a Fab structure is preferred. The first binding domain and the third domain can be fused to each other in a way, that they form a conventional immunoglobuline. Such immunoglobulin may have two heavy chains and two light chains of an immunoglobulin as described elsewhere herein.

The bispecific antibody construct used in accordance with the present invention is preferably in a format referred to as "scFv-IgAb" herein. Such an antibody construct comprises an immunoglobulin that has one scFv fragment fused to the C terminus of each of the two heavy chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL, and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 64 or 65 and 66.

The bispecific antibody construct used in accordance with the present invention may comprise an immunoglobulin that has one scFv fragment fused to the C terminus of each of the two heavy chains and to the C terminus of each of the two light chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL-VH(EGFR)-VL(EGFR), and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 68 or 65 and 68.

The bispecific antibody construct used in accordance with the present invention may be in the form of a single chain diabody. As such, the bispecific antibody construct may comprise one single polypeptide chain, in the arrangement VH(EGFR)-VL(CD16A)-VH(CD16A)-VL(EGFR). An illustrative example for such an antibody construct is shown in SEQ ID NO: 67.

In preferred embodiments, the bispecific antibody construct used in accordance with the present invention is a bispecific antibody construct comprising (a) a first binding domain which is capable of specifically binding CD16A, comprising: a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and (b) a second binding domain which is capable of specifically binding EGFR, comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In preferred embodiments, the bispecific antibody construct used in accordance with the present invention is a bispecific antibody construct comprising (a) a first binding domain comprising a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab; (b) a second binding domain comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is a bispecific antibody construct comprising (a) a first binding domain which is capable of specifically binding CD16A, comprising: a VH region as depicted in SEQ ID NO: 14 and a VL region as depicted in SEQ ID NO: 15; and (b) a second binding domain which is capable of specifically binding EGFR, comprising a VH region as depicted in SEQ ID NO: 61 and a VL region as depicted in SEQ ID NO: 62.

In preferred embodiments, the bispecific antibody construct used in accordance with the present invention is a bispecific antibody construct comprising (a) a first binding domain comprising a VH region as depicted in SEQ ID NO: 14 and a VL region as depicted in SEQ ID NO: 15, wherein said first binding domain is a Fab; (b) a second binding domain comprising a VH region as depicted in SEQ ID NO: 61 and a VL region as depicted in SEQ ID NO: 62, wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64.

In preferred embodiments, the bispecific antibody construct used in accordance with the present invention is an antibody construct comprising or consisting of amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences set forth in SEQ ID NOs: 63 and 64. In this respect is envisaged that the bispecific antibody construct is preferably AFM24.

The term "taxane" as used herein refers to the taxanes used as chemotherapeutics, e.g. in tumor treatment. Taxanes are a class of diterpenes. They were originally identified from plants of the genus Taxus (yews), and feature a taxadiene core. E.g. paclitaxel (Taxol) and docetaxel (Taxotere) are widely used as chemotherapy agents. Cabazitaxel was FDA approved to treat hormone-refractory prostate cancer.

It was found that Docetaxel in combination with a PD-1 inhibitor showed increased response rates and progression fee survival (PFS) as compared to docetaxel alone; and historically compared to a PD-1 inhibitor alone, leading to a synergistic effect (Arrieta O, Barrón F, Ramírez-Tirado LA, Zatarain-Barrón ZL, Cardona AF, Diaz-Garcia D, Yamamoto Ramos M, Mota-Vega B, Carmona A, Peralta Alvarez MP, Bautista Y, Aldaco F, Gerson R, Rolfo C, Rosell R. Efficacy and Safety of Pembrolizumab Plus Docetaxel vs Docetaxel Alone in Patients With Previously Treated Advanced Non-Small Cell Lung Cancer: The PROLONG Phase 2 Randomized Clinical Trial. JAMA Oncol. 2020 Jun 1;6(6):856-864).

In one embodiment, the taxanes used in the invention may be selected from the group consisting of docetaxel, paclitaxel, cabazitaxel.

In one preferred embodiment, the taxane used in the invention is docetaxel.

Immune checkpoint inhibitors (ICIs) can be used to treat many types of cancers, including non-small cell lung cancer, melanoma, renal cell carcinoma, head and neck cancer, and gastrointestinal cancer. ICIs function to block the inactivity of immune cells towards tumor cells. Normally, immune cells are controlled under homeostatic conditions using immune checkpoints which maintain a balance between pro-inflammatory and anti-inflammatory signals. ICIs can promote anti-cancer immune responses, shifting the balance to proinflammatory, by binding to immune inhibitory receptors, such as CTLA-4, PD-1, and PD-L receptors on the surface of T-lymphocytes, and allowing activation of the T-lymphocytes towards the tumor. By blocking the immune inhibitory receptors, the ICIs overcome tumor-mediated immune inhibition, and facilitate a local inflammatory anti-tumor effect.

Generally, an immune checkpoint inhibitor that can be used in accordance with the present invention can be one that binds to CTLA-4, PD-1, or PD-L receptors. In some examples, such an immune checkpoint inhibitor binds to a receptor on the surface of T-lymphocytes, and allowing activation of the T-lymphocytes towards the tumor. Preferably, an immune checkpoint inhibitor that can be used in accordance to the present disclosure is one that targets PD-1/PD-L1, which are immune-checkpoint molecules that inhibit the activation of antigen-presenting cells and T cells (priming phase) and the cytotoxic functions of T cells (effector phase). Preferred immune checkpoint inhibitors are PD-L1 inhibitors and PD-1 inhibitors, with PD-L1 inhibitors being more preferred. The immune checkpoint inhibitor is preferably an antibody or an antibody construct. Hence, preferred immune checkpoint inhibitors include anti-PD-1 antibodies and anti-PD-L1 antibodies. Most preferred are anti-PD-L1 antibodies.

It is envisioned by the present disclosure, that the immune checkpoint inhibitor may be an antibody that comprises means (e.g., an antigen binding site) for specifically binding PD-1 or PD-L1, preferably PD-L1.

Several anti-PD-1 antibodies and anti-PD-L1 antibodies are known to the skilled person and their use according to the present disclosure is contemplated. Examples of such antibodies are given in the following. Nivolumab (OPDIVO^{™}, Bristol Myers Squibb) is a human IgG4 monoclonal antibody that blocks PD-1. See WO2006121168A1 (Ono Co. LTD.). Atezolizumab (Tecentriq^{™}, Genetech) is a humanized, monoclonal antibody of IgG1 isotype against PD-L1 and is used for the treatment of various cancer types. See WO2010077634A1 (Genetech). Pembrolizumab (Keytruda^{™}, Merck), is a humanized IgG4 isotype anti antibody against the PD-1 receptor. See WO2008156712 A1 (N. V. Organon). Cemiplimab (Libtayo^{™}, Regeneron s, Inc.) is a monoclonal antibody that binds to PD-1. See WO2015112800A1, H4H7798N (Regeneron s, Inc). Dostarlimab (TSR-042, JEMPERLI^{™}, Glaxo Smith Kline) is a humanized, monoclonal antibody of the IgG 4κ isotype that binds to PD-1. Durvalumab (Imfinzi^{™}, Medimmune/AstraZeneca) is a human immunoglobulin G1 kappa (IgG1κ) monoclonal antibody that blocks the interaction of PD-L1 with PD-1.

Other anti-PD-1 antibodies include pidilizumab, MED10608, BI 754091, PF-0680159, spartalizumab, camrelizumab, JNJ-63723283, and MCLA-134.

Other anti-PD-L1 antibodies include H2M8314N, avelumab, MDX-1105, LY3300054, FAZ053, STI-1014, CX-072, KN035, and CK-301.

An immune checkpoint inhibitor that can be used in accordance with the present invention, may be selected from the group consisting of nivolumab, atezolizumab, pembrolizumab cemiplimab, H4H7798N, dostarlimab, durvalumab, pidilizumab, MED 10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301. Preferably, the immune checkpoint inhibitor is selected from the group consisting of nivolumab atezolizumab, pembrolizumab cemiplimab, H4H7798N, dostarlimab, and durvalumab. More preferably, the immune checkpoint inhibitor is selected from the group consisting of nivolumab atezolizumab and pembrolizumab, most preferably the immune checkpoint inhibitor is atezolizumab.

In a preferred embodiment, the bispecific antibody construct is AFM24, the taxane is docetaxel and the immune checkpoint inhibitor is atezolizumab.

The terms "subject," "individual," or "patient" are often used interchangeably herein. Subjects "suffering from a disease" or those "in need of treatment" include those already with the disorder or disease, as well as those in which onset or reoccurrence of the disorder or disease, or symptoms thereof as described elsewhere herein, is to be prevented or delayed. Suitable patients for the means and methods described herein include those who are suffering from, who have been diagnosed with, or who are suspected of having tumor disease.

The terms "subject in need" or those "in need of treatment" includes those already with the disorder or disease, as well as those in which the disorder or disease is to be prevented. The subject in need or "patient" includes human and other mammalian subjects that receive either prophylactic or therapeutic treatment.

The term "*in vivo*" is used to describe an event that takes place in a subject's body. The term "*ex vivo*" is used to describe an event that takes place outside of a subject's body. An *ex vivo* assay is not performed on a subject. Rather, it is performed upon a sample separate from a subject. An example of an *ex vivo* assay performed on a sample is an "*in vitro*" assay. The term "*in vitro*" is used to describe an event that takes places contained in a container for holding laboratory reagent such that it is separated from the biological source from which the material is obtained. *In vitro* assays can encompass cell-based assays in which living or dead cells are employed. *In vitro* assays can also encompass a cell-free assay in which no intact cells are employed.

In some embodiments, the subject is a mammal selected from the group consisting of an armadillo, an ass, a bat, a bear, a beaver, a cat, a chimpanzee, a cow, a coyote, a deer, a dog, a dolphin, an elephant, a fox, a panda, a gibbon, a giraffe, a goat, a gopher, a hedgehog, a hippopotamus, a horse, a humpback whale, a jaguar, a kangaroo, a koala, a leopard, a lion, a llama, a lynx, a mole, a monkey, a mouse, a narwhal, an orangutan, an orca, an otter, an ox, a pig, a polar bear, a porcupine, a puma, a rabbit, a raccoon, a rat, a rhinoceros, a sheep, a squirrel, a tiger, a walrus, a weasel, a wolf, a zebra, a goat, a horse, and combinations thereof. In some embodiments, the mammal is a human. Thus, subjects to be treated using the means and methods provided herein include human and other mammalian subjects, who have been diagnosed with or are suspected of having a tumor disease. In some embodiments, the subject may be a human who exhibits one or more symptoms associated with a tumor disease.

The subject, e.g., the human subject, can be a child, e.g., from or from about 0 to or to about 14 years in age. The subject can be a youth, e.g., from or from about 15 to or to about 24 years in age. The subject can be an adult, e.g., from or from about 25 to or to about 64 years in age. The subject can be a senior, e.g. 65+ years in age.

In some embodiments, the subject may also be one that shows satisfactory results at the onset of an initial treatment, but becomes less responsive to said treatment after a period of time. Subjects having received previous treatments may have undergone treatments with different drugs, or drug combinations, at different times. For example, some subjects may have undergone two or more therapies using different drugs of drug combinations, wherein the therapies were discontinued as not providing desired results.

Subjects that can be treated using the means and methods described herein include those subjects that have undergone "previous treatment". "Previous treatment" as used herein refers to treatment of a tumor that occurs prior to the treatment provided by the present invention. For example, the subject may have been diagnosed to suffer from NSCLC or any other solid tumor. After the previous treatment of NSCLC, the subject may not show satisfactory results, such as a partial unsatisfactory response to treatment.

Previous treatment of said subject may also comprise administration of at least an immune checkpoint inhibitor (ICI) as described elsewhere herein. After the previous treatment, the subject may not show satisfactory results, such as a partial unsatisfactory response to the ICI treatment, or no observable response to the ICI treatment. The subject may also be one that shows satisfactory results at the onset of treatment, but that becomes less responsive to the treatment after a period of time. The previous ICI treatment can use an ICI that binds to immune inhibitory receptors, such as CTLA-4, PD-1, and PD-L receptors. For example, the subject may have had a previous ICI treatment with an immune checkpoint inhibitor such as ipilimumab, pembrolizumab, nivolumab, cemiplimab, dostarlimab, atezolizumab, durvalumab, and avelumab. See, for example, Lee, J.B., et al. (2022) Immune Checkpoint Inhibitors in 10 Years: Contribution of Basic Research and Clinical Application in Cancer Immunotherapy. Immune Netw. 22(1):e2.

As set forth above, the subject receiving previous treatment may have also been treated with one or more traditional chemotherapeutic drugs. For example, the previous treatment may have used one or more platinum-based drugs such as cisplatin, carboplatin, and oxaliplatin. Patients previously treated with platinum-based drugs may have had their treatment discontinued due to systemic toxicity and drug resistance.

The subject receiving previous treatment may have also been treated with one or members of the taxane class of drugs such as paclitaxel (taxol), docetaxel (taxotere), and cabazitaxel. These types of drugs disrupt microtubule function, which are critical to cell division, and inhibit the process of cell division by preventing as depolymerization. These drugs are also widely used to treat a variety of solid tumors, such as NSCLC. Patients previously treated with taxane class of drugs may have had their treatment discontinued due to toxicities, such as neutropenia, neuropathy, hypersensitivity, and alopecia.

The subject receiving previous treatment may have also been treated with one or more antimetabolite drugs such as methotrexate, pemetrexed, raltitrexed and pralatrexate. Patients previously treated with taxane class of drugs may have had their treatment discontinued due to side effects such as low blood cell count, fatigue, nausea, and gastrointestinal problems.

In some embodiments, the subject may also be one that shows satisfactory results at the onset of an initial treatment, but becomes less responsive to said treatment after a period of time. Subjects having received previous treatments may have undergone treatments with different drugs, or drug combinations, at different times. For example, some subjects may have undergone two or more therapies using different drugs of drug combinations, wherein the therapies were discontinued as not providing desired results.

Any of the means and methods of treatment provided herein may be used to treat a tumor disease at various stages. By way of example, the cancer stage includes but is not limited to early, advanced, locally advanced, remission, refractory, reoccurred after remission and progressive. In some embodiments, the subject is at an early stage of a cancer. In other embodiments, the subject is at an advanced stage of cancer. In various embodiments, the subject has a stage I, stage II, stage III or stage IV cancer. The means and methods described herein can promote reduction or retraction of a tumorous cells, decrease or inhibit cancer cell proliferation, and/or induce, increase or promote tumor cell killing. In some embodiments, the subject is in cancer remission. The means and methods described herein can prevent or delay metastasis or recurrence of cancer.

In certain embodiments, the subject may be a human who is (i) substantially refractory to at least one chemotherapy treatment, or (ii) is in relapse after treatment with chemotherapy, or both (i) and (ii). In some of embodiments, the subject is refractory to at least two, at least three, or at least four chemotherapy treatments (including standard or experimental chemotherapies described elsewhere herein).

In some embodiments, the subject has relapsed after treatment with or is refractory to an antibody therapy as described e.g. in Morse at al. 2023, Expert Opinion on Investigational Drugs, 32 (2): 107-125. In some embodiments, the subject is refractory to or has a recurrence after treatment with a tyrosine kinase inhibitor. In some embodiments, the tyrosine kinase inhibitor is selected from the group consisting of gefitinib, erlontinib, afatinib, osimertinib, and combinations thereof.

In the context of the present invention, responsiveness to therapy can generally determined by measuring or determining the amount of EGFR-positive non-small lung cancer cells in a sample obtained from the treated subject, e.g. a sample obtained by lung biopsy. A high responsiveness of a patient to treatment described herein means that a significantly reduced amount of EGFR-positive non-small lung cancer cells can be detected in said sample obtained from the treated subject. A low responsiveness of a patient to treatment described herein means that no significantly reduced amount of EGFR-positive non-small lung cancer cells or even an increase of the amount of EGFR-positive non-small lung cancer cells can be detected in a sample obtained from the treated subject when compared to the amount of EGFR-positive non-small lung cancer cells obtained from said patient before the respective treatment. The responsiveness of tumors is objectively assessed according to the "Response Evaluation Criteria in Solid Tumors" (RECIST).

Alternative to lung biopsy, responsiveness of NSCLC patients to therapy can also be determined using methods such as chest x-ray (CXR), computed tomography (CT) scan, magnetic resonance imaging (MRI), positron emission tomography (PET) scan, or sputum analysis, known to those skilled in the art, thereby comparing the results before the respective treatment with the results obtained after the respective treatment. Accordingly, responsiveness to treatment can be determined *in vitro* or *in vivo.*

As set forth above, in the context of the present invention, the bispecific antibody construct described herein, e.g. a pharmaceutical compositions comprising the bispecific antibody construct described herein, is administered to the subject in combination with a taxane as described herein, e.g., a pharmaceutical composition comprising said taxane as described herein, and optional in further combination with an immune checkpoint inhibitor as described herein, e.g., a pharmaceutical composition comprising said immune checkpoint inhibitor as described herein. In some embodiments, the bispecific antibody construct is administered together with the taxane and/or together with the immune checkpoint inhibitor as part of one pharmaceutical composition and/or combination. In some embodiments, the bispecific antibody construct may be administered separately from the taxane and/or separately from the immune checkpoint inhibitor, e.g., as part of a separate pharmaceutical composition. According to this embodiment, also the taxane may be administered separately from the immune checkpoint inhibitor e.g., as part of a separate pharmaceutical composition. The bispecific antibody constructs comprising at least a CD16A and an EGFR binding domain as described herein can be administered prior to, subsequent to, or simultaneously with administration of the taxane and/or the immune checkpoint inhibitor. In some embodiments, the taxane can be administered prior to, subsequent to, or simultaneously with administration of the bispecific antibody construct and/or the immune checkpoint inhibitor. In some embodiments, the immune checkpoint inhibitor may be administered prior to, subsequent to, or simultaneously with administration of the taxane and/or the bispecific antibody construct.

Therapeutics used in the treatment of cancer are delivered to the subject usually in a specific dosing scheme.

The bispecific antibody construct, as part of the invention, may be administered to the subject once every about 6 to about 8 days, or once every about 7 days.

The bispecific antibody construct may be administered at a dose in the range of about 300 to about 800 mg, about 350 to about 700 mg, about 400 to about 550 mg, about 420 to about 530 mg, about 450 to about 500 mg, or about 480 mg.

The taxanes, as part of the invention, may be administered to the subject once every about 2 to about 4 weeks, once every about 3 weeks, once every about 19 to about 23 days, once every about 20 to about 22 days, or once every about 21 days.

The taxane may be administered at a dose in the range of about 60 to about 90 mg/m², about 70 to about 80 mg/m², about 72 to about 78 mg/m², or about 75 mg/m².

The immune checkpoint inhibitor may be administered to the subject once every about 2 to about 4 weeks, once every about 3 weeks, once every about 19 to about 23 days, once every about 20 to about 22 days, or once every about 21 days.

The immune checkpoint inhibitor may be administered at a dose in the range of about 1000 to about 1400 mg, about 1100 to about 1300 mg, about 1150 to about 1250 mg, or about 1200 mg.

Chemotherapeutics are usually administered in a treatment cycle.

In some embodiments, the bispecific antibody construct as described herein, the immune checkpoint inhibitor as described herein and/or the taxane as described herein are administered to the subject as part of a treatment cycle that spans multiple days. In some embodiments, the bispecific antibody construct, the taxane and/or the immune checkpoint inhibitor are administered to the subject as part of a treatment regimen that comprises one or more treatment cycles.

In some embodiments, one treatment cycle is from 2 to 60 days, e.g., from 2 to 50, 2 to 40, 2 to 30, 2 to 20, 2 to 10, 2 to 5, 5 to 60, 5 to 50, 5 to 40, 5 to 30, 5 to 20, 5 to 10, 10 to 60, 10 to 50, 10 to 40, 10 to 30, 10 to 20, 20 to 60, 20 to 50, 20 to 40, 20 to 30, 30 to 60, 30 to 50, 30 to 40, 40 to 60, 40 to 50, or 50 to 60 days. In some embodiments, the treatment cycle is from 1 to 8 weeks (e.g., 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks). In some embodiments, the treatment cycle is or is about 1, 2, 3, 4, 5, 6, 7, or 8 weeks. Preferably one treatment cycle may last about 3 weeks or 21 days.

In some embodiments, the treatment regimen may comprise a treatment break (e.g., a period without administration of the bispecific antibody construct, of the taxane, and/or of the immune checkpoint inhibitor) between treatment cycles. In some embodiments, the treatment break is from 1 to 8 weeks, e.g., from 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks. In some embodiments, the treatment break is at least 1, 2, 3, 4, 5, 6, 7, or 8 weeks. In some embodiments, the treatment break is or is about 1, 2, 3, 4, 5, 6, 7, or 8 weeks.

In some embodiments, one treatment cycle may consist of administering the bispecific antibody construct 3 times, administering the taxane 1 time, and administering the immune checkpoint inhibitor 1 time. The treatment of the subject is carried out for at least one cycle.

In some embodiments, the administration of the taxane and of the immune checkpoint inhibitor may be separated by a time period of at least about 5 days, at least about 6 days or at least about 7 days. The taxane may be administered in the first week of each cycle.

A dose of the taxane and a dose of the bispecific antibody construct could be administered to the subject at the same time, or about the same time. Furthermore, the dose the taxane could be concurrently administered with a dose of the bispecific antibody construct. This shortens the total time of administration of the therapeutics. It is further possible to administer a dose of the bispecific antibody molecule and/or a dose the taxane as split-day dosing on two consecutive days. It is also possible to administer the first dose of the bispecific antibody molecule and the dose of taxane during the first cycle as split-day dosing on two consecutive days.

In some embodiments, the immune checkpoint inhibitor may be administered in the second week of each cycle.

A dose the immune checkpoint inhibitor and a dose of the bispecific antibody construct may be administered to the subject at the same time, or about the same time. The dose the immune checkpoint inhibitor could be concurrently administered with a dose of the bispecific antibody construct for shortening the total time needed for administration.

As part of the dosing scheme mentioned above a special lead-in dose of the bispecific antibody may be used. Such a lead-in dose of the bispecific antibody construct could be administered to the subject about 5 to about 8 days or about 6 to about 7 days prior to a first cycle of administration. The lead-in dose may have a range of about 400 to about 550 mg, about 420 to about 530 mg, about 450 to about 500 mg, or about 480 mg. Furthermore, the lead-in dose may be administered as a split-dose on two consecutive days.

The bispecific antibody construct, the taxane, and/or the immune checkpoint inhibitor may be administered intravenously.

In some embodiments, the treatment regimen comprises from 1 to 5 treatment cycles, e.g., 1 to 4, 1 to 3, 1 to 2, 2 to 5, 2 to 4, 2 to 3, 3 to 5, 3 to 4, or 4 to 5 treatment cycles. In some embodiments, the treatment regimen comprises 1, 2, 3, 4, or 5 treatment cycles. In some embodiments, the treatment cycles of a treatment regimen are the same (e.g., follow the same dosing and timing schedules). In some embodiments, the treatment cycles of a treatment regimen are different (e.g., follow different dosing and timing schedules).

In some embodiments, the treatment regime comprises the administration of the bispecific antibody construct, the taxane, and/or the immune checkpoint inhibitor to be carried out for a period of time in the range of 2 weeks to 2 years, or 1 month to 18 months, or 3 months to 12 months.

In some embodiments, the treatment regimen continues until the subject's disorder (i.e. the tumor disease) progresses, or until the doses are discontinued due to the subject's intolerance of the bispecific antibody construct, of the taxane, of the immune checkpoint inhibitor, or due to the patient's intolerance of at least two of them, or due to the subject's intolerance of all three compounds.

Furthermore, after each cycle of treatment the tumor regression may be assessed.

In a preferred embodiment, the bispecific antibody construct, taxane, and/or immune checkpoint inhibitor for the use of any one of the preceding claims, wherein the bispecific antibody construct, preferably AFM24, is administered to the subject on day 1, 8, and 15 of each 21-day cycle at a dose of 480 mg weekly; and the taxane, preferably docetaxel, is administered to the subject on day 1 of each 21-day cycle at a dose of 75 mg/m² every 3 weeks (q3w); and the immune checkpoint inhibitor, preferably atezolizumab, is administered to the subject on day 8 of each 21-day cycle at a dose of 1200 mg q3w; wherein the bispecific antibody construct and the taxanes is administered to the subject on day 1 of the first and second cycle as a split day-dose on day 1 and 2 and wherein the treatment lasts up to 24 months or disease progression, optionally wherein a lead-in dose of the bispecific antibody construct, preferably AFM24, is administered to the subject as a split-day dose on days 7 and 6 prior to the first cycle in an amount of 480 mg.

The results of the inventive tumor treatment could be measured in different ways. One result of the treatment may be the regression or shrinkage of the tumor. Further independent results could be changes in the tumor microenvironment or the expression of specific molecules by the cancer cells or the cells surrounding the cancer cells.

The inventive treatment may result in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.

The inventive treatment including a taxane may further result in a reduction of the number of M2 macrophages in the tumor microenvironment as compared to a treatment regimen comprising the administration of the bispecific antibody molecule and the immune checkpoint inhibitor, but without the administration of a taxanes.

The inventive treatment including a taxane may also inhibits reduce, or revert the transformation of a tumor microenvironment to an immune-suppressive tumor-microenvironment as compared to a treatment regimen comprising the administration of the bispecific antibody molecule and the immune checkpoint inhibitor, but without the administration of a taxane.

Furthermore, the inventive treatment of the tumor may repolarize M2 macrophages in the tumor microenvironment to M1 macrophages, wherein the M2 macrophages are associated with the treatment of a tumor with the bispecific antibody construct.

The invention relates also to a pharmaceutical composition comprising a taxane and a bispecific antibody construct. The antibody construct comprises at least one first binding domain capable of specifically binding CD16A and at least one second binding domain capable of specifically binding EGFR.

In this pharmaceutical composition the taxane may be present in an amount (by weight) that is in the range of about 5 to about 8 times lower than an amount of the bispecific antibody construct.

The pharmaceutical composition may in a liquid form and configured for intravenous (iv) injection. Therefore, the composition may comprise one or more excipients.

This pharmaceutical composition should be for use in a method of treating a tumor in a subject. Also, a method of treatment of a tumor in a subject is contemplated using said composition.

In a further embodiment the invention relates to a use of said pharmaceutical composition for the manufacture of a medicament for the treatment of a subject.

The pharmaceutical composition described before could also be used in the method of treating a tumor described above with regard to a bispecific antibody construct, a taxane, and/or an immune checkpoint inhibitor. Thus, all the features mentioned above of to the bispecific antibody construct and the taxane including the dosing schemes and the results also apply to the composition comprising the taxane and the bispecific antibody construct.

The term "pharmaceutical composition" relates to a composition which is suitable for administration to a subject, preferably a human subject. In some embodiments, the pharmaceutical composition administered to said subject comprises one or a plurality of the bispecific antibody construct(s) described. Accordingly, in the context of the present invention, the bispecific antibody construct(s) described herein may be comprised in a first pharmaceutical composition and the taxane described herein may be comprised in a second pharmaceutical composition, and the immune checkpoint inhibitor described herein may be comprised in a third pharmaceutical composition which may all be administered to said patient in the course of the therapeutic treatment. In some embodiments, the taxane and the bispecific antibody construct(s) may be comprised in one pharmaceutical composition that is administered to said subject in the course of the therapeutic treatment. In some embodiments, the immune checkpoint inhibitor and the bispecific antibody construct(s) may be comprised in one pharmaceutical composition that is administered to said subject in the course of the therapeutic treatment. In some embodiments, the immune checkpoint inhibitor and the taxane may be comprised in one pharmaceutical composition that is administered to said subject in the course of the therapeutic treatment. In some embodiments, the taxane, the bispecific antibody construct(s), and the immune checkpoint inhibitor may be comprised in one pharmaceutical composition that is administered to said subject in the course of the therapeutic treatment. The same is true for a combination comprising a taxane, a bispecific antibody construct that comprises at least one first binding domain capable of specifically binding CD16A and at least one second binding domain capable of specifically binding EGFR, and optionally an immune checkpoint inhibitor.

Preferably, the pharmaceutical composition(s) used in the context of the present invention comprises suitable formulations of one or more (pharmaceutically effective) carriers, stabilizers, excipients, diluents, solubilizers, surfactants, emulsifiers, preservatives and/or adjuvants. Acceptable constituents of the composition are preferably nontoxic to recipients at the dosages and concentrations employed. Pharmaceutical compositions that can be used in the context of the present invention include, but are not limited to, liquid, frozen, and lyophilized compositions. As used herein the language "pharmaceutically acceptable carrier" includes saline, solvents, dispersion media, coatings, antibacterial and antifungal agents, isotonic and absorption delaying agents, and the like, compatible with pharmaceutical administration. The use of such media and agents in pharmaceutical compositions is well known in the art, and the compositions comprising such carriers can be formulated by well-known conventional methods.

Pharmaceutical compositions are typically formulated to be compatible with its intended route of administration. Examples of routes of administration include parenteral, e.g., intravenous, intradermal, subcutaneous, oral, transdermal (topical), transmucosal, and rectal administration. In the context of the means and methods of the present invention it is particularly envisaged that the pharmaceutical composition(s) comprising the taxane, and/or the bispecific antibody construct, and/or the immune checkpoint inhibitor is suitable for intravenous administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions and/or combination suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin

In certain embodiments, the pharmaceutical composition and/or combination may contain formulation materials for the purpose of modifying, maintaining or preserving, e.g., the pH, osmolarity, viscosity, clarity, color, isotonicity, odor, sterility, stability, rate of dissolution or release, adsorption or penetration of the composition (see, REMINGTON'S SCIENCES, 18" Edition, (A.R. Genrmo, ed.), 1990, Mack Publishing Company). In such embodiments, suitable formulation materials may include, but are not limited to: amino acids such as glycine, alanine, glutamine, asparagine, threonine, proline, 2- phenylalanine, including charged amino acids, preferably lysine, lysine acetate, arginine, glutamate and/or histidine; antimicrobials such as antibacterial and antifungal agents; antioxidants such as ascorbic acid, methionine, sodium sulfite or sodium hydrogen- sulfite; buffers, buffer systems and buffering agents which are used to maintain the composition at physiological pH or at a slightly lower pH; examples of buffers are borate, bicarbonate; Tris-HCI, citrates, phosphates or other organic acids, succinate, phosphate, and histidine; for example Tris buffer of about pH 7.0-8.5; non-aqueous solvents such as propylene glycol, polyethylene glycol, vegetable oils such as olive oil, and injectable organic esters such as ethyl oleate; aqueous carriers including water, alcoholic/aqueous solutions, emulsions or suspensions, including saline and buffered media; biodegradable polymers such as polyesters; bulking agents such as mannitol or glycine; chelating agents such as ethylenediamine tetraacetic acid (EDTA); isotonic and absorption delaying agents; complexing agents such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); carbohydrates may be non-reducing sugars, preferably trehalose, sucrose, octasulfate, sorbitol or xylitol; (low molecular weight) proteins, polypeptides or proteinaceous carriers such as human or bovine serum albumin, gelatin or immunoglobulins, preferably of human origin; coloring and flavouring agents; sulfur containing reducing agents, such as glutathione, thioctic acid, sodium thioglycolate, thioglycerol, [alpha]-monothioglycerol, and sodium thio sulfate; diluting agents; emulsifying agents; hydrophilic polymers such as polyvinylpyrrolidone); salt-forming counterions such as sodium; preservatives such as antimicrobials, anti-oxidants, chelating agents, inert gases and the like; examples are: benzalkonium chloride, benzoic acid, salicylic acid, thimerosal, phenethyl alcohol, methylparaben, propylparaben, chlorhexidine, sorbic acid or hydrogen peroxide); metal complexes such as Zn-protein complexes; solvents and co-solvents (such as glycerin, propylene glycol or polyethylene glycol); sugars and sugar alcohols, such as trehalose, sucrose, octasulfate, mannitol, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; and polyhydric sugar alcohols; suspending agents; surfactants or wetting agents such as pluronics, PEG, sorbitan esters, polysorbates such as polysorbate 20, polysorbate, triton, tromethamine, lecithin, cholesterol, tyloxapal; surfactants may be detergents, preferably with a molecular weight of >1.2 KD and/or a polyether, preferably with a molecular weight of >3 KD; non-limiting examples for preferred detergents are Tween 20, Tween 40, Tween 60, Tween 80 and Tween 85; non-limiting examples for preferred polyethers are PEG 3000, PEG 3350, PEG 4000 and PEG 5000; stability enhancing agents such as sucrose or sorbitol; tonicity enhancing agents such as alkali metal halides, preferably sodium or potassium chloride, mannitol sorbitol; parenteral delivery vehicles including sodium chloride solution, Ringer's dextrose, dextrose and sodium chloride, lactated Ringer's, or fixed oils; intravenous delivery vehicles including fluid and nutrient replenishers, electrolyte replenishers (such as those based on Ringer's dextrose).

It is evident to those skilled in the art that the different constituents of the pharmaceutical composition and/or combination (e.g., those listed above) can have different effects, for example, and amino acid can act as a buffer, a stabilizer and/or an antioxidant; mannitol can act as a bulking agent and/or a tonicity enhancing agent; sodium chloride can act as delivery vehicle and/or tonicity enhancing agent; etc.

It is envisaged that the pharmaceutical composition and/or combination of the invention might comprise, in addition to the polypeptide of the invention defined herein, further biologically active agents, depending on the intended use of the composition.

In certain embodiments, the optimal composition will be determined by one skilled in the art depending upon, for example, the intended route of administration, delivery format and desired dosage. See, for example, REMINGTON'S SCIENCES, supra. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles.

Additional compositions will be evident to those skilled in the art, including formulations involving the antibody construct of the invention in sustained- or controlled-delivery / release formulations. Techniques for formulating a variety of other sustained- or controlled-delivery means, such as liposome carriers, bio-erodible microparticles or porous beads and depot injections, are also known to those skilled in the art. See, for example, International Patent Application No. PCT/US93/00829, which describes controlled release of porous polymeric microparticles for delivery of compositions. Sustained-release preparations may include semipermeable polymer matrices in the form of shaped articles, e.g., films, or microcapsules. Sustained release matrices may include polyesters, hydrogels, polylactides (as disclosed in U.S. Pat. No. 3,773,919 and European Patent Application Publication No. EP 058481), copolymers of L-glutamic acid and gamma ethyl-L-glutamate (Sidman et al., 1983, Biopolymers 2:547-556), poly (2-hydroxyethylmethacrylate) (Langer et al., 1981, J. Biomed. Mater. Res. 15:167-277 and Langer, 1982, Chem. Tech. 12:98-105), ethylene vinyl acetate (Langer et al., 1981, supra) or poly-D(-)-3-hydroxybutyric acid (European Patent Application Publication No. EP 133,988). Sustained release compositions may also include liposomes that can be prepared by any of several methods known in the art. See, e.g., Eppstein et al., 1985, Proc. Natl. Acad. Sci. U.S.A. 82:3688-3692; European Patent Application Publication Nos. EP 036,676; EP 088,046 and EP 143,949.

The bispecific antibody construct may also be entrapped in microcapsules prepared, for example, by coacervation techniques or by interfacial polymerization (for example, hydroxymethylcellulose or gelatine-microcapsules and poly (methylmethacylate) microcapsules, respectively), in colloidal drug delivery systems (for example, liposomes, albumin microspheres, microemulsions, nanoparticles and nanocapsules), or in macroemulsions. Such techniques are disclosed in Remington's Sciences, 16th edition, Oslo, A., Ed., (1980).

The pharmaceutical composition and/or combination used for in vivo administration are typically provided as sterile preparations. Sterilization can be accomplished by filtration through sterile filtration membranes. When the pharmaceutical composition is lyophilized, sterilization using this method may be conducted either prior to or following lyophilization and reconstitution. Pharmaceutical compositions for parenteral administration can be stored in lyophilized form or in a solution. Parenteral pharmaceutical compositions generally are placed into a container having a sterile access port, for example, an intravenous solution bag or vial having a stopper pierceable by a hypodermic injection needle.

Sterile injectable solutions can also be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

The invention also relates to a combination comprising a taxane, a bispecific antibody construct that comprises at least one first binding domain capable of specifically binding CD16A and at least one second binding domain capable of specifically binding EGFR, and optionally an immune checkpoint inhibitor.

The combination should be for use in a method of treating a tumor in a subject. Also, a method of treatment of a tumor in a subject is contemplated using said combination. Further, the use of said combination for the manufacture of a medicament for the treatment of a subject is contemplated.

The combination described before could also be used in the method of treating a tumor described above with regard to a bispecific antibody construct, a taxane, and/or an immune checkpoint inhibitor. Thus, all the features mentioned above of to the bispecific antibody construct and the taxane including the dosing schemes and the results also apply to the composition comprising the taxane and the bispecific antibody construct.

The invention further relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of M2 macrophages in a tumor microenvironment associated with the treatment of a tumor with a bispecific antibody construct and optionally an immune checkpoint inhibitor.

The invention further relates to a method of treatment of a tumor by inhibiting, reducing, or reverting the increase of M2 macrophages in a tumor microenvironment associated with the treatment of a tumor with a bispecific antibody construct and optionally an immune checkpoint inhibitor using a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR.

The invention also relates to a use of a taxane, a bispecific antibody construct, or a combination of a taxane, a bispecific antibody construct and optionally an immune checkpoint inhibitor for the manufacture of a medicament for the treatment of a tumor in a subject, wherein the bispecific antibody construct comprises at least one first binding domain capable of specifically binding CD16A and at least one second binding domain capable of specifically binding EGFR, and wherein the treatment may result in inhibiting, reducing, or reverting the increase of M2 macrophages in a tumor microenvironment associated with the treatment of a tumor with a bispecific antibody construct and optionally an immune checkpoint inhibitor.

The taxane, the bispecific antibody construct, or the combination of the taxane, the bispecific antibody construct and optionally the immune checkpoint inhibitor described in the three paragraphs before could also be used in the method of treating a tumor described above with regard to a bispecific antibody construct, a taxane, and/or an immune checkpoint inhibitor. Thus, all the features mentioned above of to the bispecific antibody construct, the taxane, and/or the immune checkpoint inhibitor including the dosing schemes and the results also apply to the composition comprising the taxane and the bispecific antibody construct.

The invention further relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor.

The invention also relates to a method of treatment of a tumor in a subject by inhibiting, reducing, or reverting the transformation of a tumor microenvironment to an immune-suppressive tumor-microenvironment associated with the treatment of a tumor with a bispecific antibody construct and optionally an immune checkpoint inhibitor using a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR.

The invention also relates to a use of a taxane, a bispecific antibody construct, or a combination of a taxane, a bispecific antibody construct and optionally an immune checkpoint inhibitor for the manufacture of a medicament for the treatment of a tumor in a subject, wherein the bispecific antibody construct comprises at least one first binding domain capable of specifically binding CD16A and at least one second binding domain capable of specifically binding EGFR and wherein the treatment may result in inhibiting, reducing, or reverting the transformation of a tumor microenvironment to an immune-suppressive tumor-microenvironment associated with the treatment of a tumor with a bispecific antibody construct and optionally an immune checkpoint inhibitor.

The taxane, the bispecific antibody construct, or the combination of the taxane, the bispecific antibody construct and optionally the immune checkpoint inhibitor described in the three paragraphs before could also be used in the method of treating a tumor described above with regard to a bispecific antibody construct, a taxane, and/or an immune checkpoint inhibitor. Thus, all the features mentioned above of to the bispecific antibody construct, the taxane, and/or the immune checkpoint inhibitor including the dosing schemes and the results also apply to the composition comprising the taxane and the bispecific antibody construct.

The invention further relates to a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of repolarizing M2 macrophages in the tumor microenvironment to M1 macrophages, wherein the M2 macrophages are associated with the treatment of a tumor with a bispecific antibody construct and optionally an immune checkpoint inhibitor.

The invention also relates to a method of treatment of tumor in a subject by repolarizing M2 macrophages in the tumor microenvironment to M1 macrophages, wherein the M2 macrophages are associated with the treatment of a tumor with a bispecific antibody construct and optionally an immune checkpoint inhibitor using a taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR.

The invention also relates to a use of a taxane, a bispecific antibody construct, or a combination of a taxane, a bispecific antibody construct and optionally an immune checkpoint inhibitor for the manufacture of a medicament for the treatment of a tumor in a subject by repolarizing M2 macrophages in the tumor microenvironment to M1 macrophages, wherein the M2 macrophages are associated with the treatment of a tumor with a bispecific antibody construct and optionally an immune checkpoint inhibitor, wherein the bispecific antibody construct comprises at least one first binding domain capable of specifically binding CD16A and at least one second binding domain capable of specifically binding EGFR.

The taxane, the bispecific antibody construct, or the combination of the taxane, the bispecific antibody construct and optionally the immune checkpoint inhibitor described in the three paragraphs before could also be used in the method of treating a tumor described above with regard to a bispecific antibody construct, a taxane, and/or an immune checkpoint inhibitor. Thus, all the features mentioned above of to the bispecific antibody construct, the taxane, and/or the immune checkpoint inhibitor including the dosing schemes and the results also apply to the composition comprising the taxane and the bispecific antibody construct.

The invention is further characterized by the following items.
1. A bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.
   item
2. The bispecific antibody construct, taxane, and/or immune checkpoint inhibitor for the use of item 1, wherein the tumor is a solid tumor.
3. The bispecific antibody construct, taxane, and/or immune checkpoint inhibitor for the use of item 1 or 2, wherein the tumor characterized by epidermal growth factor receptor (EGFR) overexpression on tumor cells.
4. The bispecific antibody construct, taxane, and/or immune checkpoint inhibitor for the use of any one of the preceding items, wherein the tumor is an EGFR-positive tumor.
5. The bispecific antibody construct, taxane, and/or immune checkpoint inhibitor for the use of any one of the preceding items, wherein the tumor is selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN).
6. The bispecific antibody construct, taxane, and/or immune checkpoint inhibitor for the use of any one of the preceding items, wherein the tumor is lung cancer.
7. The bispecific antibody construct, taxane, and/or immune checkpoint inhibitor for the use of any one of the preceding items, wherein the tumor is non-small cell lung cancer (NSCLC).
8. The bispecific antibody construct, taxane, and/or immune checkpoint inhibitor for the use of any one of the preceding items, wherein the first binding domain binds to an epitope on CD16A which is C-terminal to the physiological Fcy receptor binding domain, said epitope preferably comprising Y158 of SEQ ID NO: 1.
9. The bispecific antibody construct, taxane, and/or immune checkpoint inhibitor for the use of any one of the preceding items, wherein the first binding domain comprises
   (i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
   (ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
   (iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
   (iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
   (v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).
10. The bispecific antibody construct, taxane, and/or immune checkpoint inhibitor for the use of any one of the preceding items, wherein the first binding domain comprises
   (i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
   (ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
   (iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
   (iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
   (v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
   (vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
   (vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
   (viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
   (ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55.
11. The bispecific antibody construct, taxane, and/or immune checkpoint inhibitor for the use of any one of the preceding items, wherein the first binding domain comprises
   (i) a VH region as depicted in SEQ ID NO: 14 and a VL region as depicted in SEQ ID NO: 15;
   (ii) a VH region as depicted in SEQ ID NO: 22 and a VL region as depicted in SEQ ID NO: 23;
   (iii) a VH region as depicted in SEQ ID NO: 30 and a VL region as depicted in SEQ ID NO: 31;
   (iv) a VH region as depicted in SEQ ID NO: 44 and a VL region as depicted in SEQ ID NO: 45;
   (v) a VH region as depicted in SEQ ID NO: 46 and a VL region as depicted in SEQ ID NO: 47;
   (vi) a VH region as depicted in SEQ ID NO: 48 and a VL region as depicted in SEQ ID NO: 49;
   (vii) a VH region as depicted in SEQ ID NO: 53 and a VL region as depicted in SEQ ID NO: 50;
   (viii) a VH region as depicted in SEQ ID NO: 55 and a VL region as depicted in SEQ ID NO: 51; or
   (ix) a VH region as depicted in SEQ ID NO: 52 and a VL region as depicted in SEQ ID NO: 53.
12. The bispecific antibody construct, taxane, and/or immune checkpoint inhibitor for the use of any one of the preceding items, wherein the first binding domain is a variable domain (Fv), a single chain Fv (scFv), a Fab, a single chain diabody (scDb), a diabody (Db) or a double Fab.
13. The bispecific antibody construct, taxane, and/or immune checkpoint inhibitor for the use of any one of the preceding items, wherein the second binding domain comprises a VH and a VL domain of an antibody.
14. The bispecific antibody construct, taxane, and/or immune checkpoint inhibitor for the use of any one of the preceding items, wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).
15. The bispecific antibody construct, taxane, and/or immune checkpoint inhibitor for the use of any one of the preceding items, wherein the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62.
16. The bispecific antibody construct, taxane, and/or immune checkpoint inhibitor for the use of any one of the preceding items, wherein the second binding domain comprises a VH region as depicted in SEQ ID NO: 61 and a VL region as depicted in SEQ ID NO: 62.
17. The bispecific antibody construct, taxane, and/or immune checkpoint inhibitor for the use of any one of the preceding items, wherein the second binding domain is a variable domain (Fv), a single chain Fv (scFv), a Fab, a single chain diabody (scDb), a diabody (Db) or a double Fab.
18. The bispecific antibody construct, taxane, and/or immune checkpoint inhibitor for the use of any one of the preceding items, wherein the bispecific antibody construct binds to a target cell and an immune effector cell simultaneously.
19. The bispecific antibody construct, taxane, and/or immune checkpoint inhibitor for the use of any one of the preceding items, wherein the bispecific antibody construct further comprises a third domain comprising a half-life extension domain.
20. The bispecific antibody construct, taxane, and/or immune checkpoint inhibitor for the use of any one of the preceding items, wherein said half-life extension domain comprises a CH2 domain, wherein the Fcγ receptor binding domain is silenced.
21. The bispecific antibody construct, taxane, and/or immune checkpoint inhibitor for the use of any one of the preceding items, wherein said half-life extension domain comprises a CH3 domain.
22. The bispecific antibody construct, taxane, and/or immune checkpoint inhibitor for the use of any one of the preceding items, wherein the bispecific antibody construct comprises at least one hinge domain and a CH3 domain fused to a CH2 domain in an amino to carboxyl order in the order hinge domain - CH2 domain - CH3 domain.
23. The bispecific antibody construct, taxane, and/or immune checkpoint inhibitor for the use of any one of the preceding items, wherein the antibody construct comprises at least two of the hinge domain - CH2 domain - CH3 domain elements.
24. The bispecific antibody construct, taxane, and/or immune checkpoint inhibitor for the use of any one of the preceding items, wherein the second binding domain is fused to the C terminus of a CH3 domain and the first binding domain is fused to the N terminus of a hinge region.
25. The bispecific antibody construct, taxane, and/or immune checkpoint inhibitor for the use of any one of the preceding items, wherein the antibody construct is bivalent for the first binding domain and bivalent for the second binding domain.
26. The bispecific antibody construct, taxane, and/or immune checkpoint inhibitor for the use of any one of the preceding items, wherein
   (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and
   (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).
27. The bispecific antibody construct, taxane, and/or immune checkpoint inhibitor for the use of any one of the preceding items, wherein
   (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab;
   (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and
   (c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.
28. The bispecific antibody construct, taxane, and/or immune checkpoint inhibitor for the use of any one of the preceding items, wherein
   (a) the first binding domain comprises a VH region as depicted in SEQ ID NO: 14 and a VL region as depicted in SEQ ID NO: 15; and
   (b) the second binding domain comprises a VH region as depicted in SEQ ID NO: 61 and a VL region as depicted in SEQ ID NO: 62.
29. The bispecific antibody construct, taxane, and/or immune checkpoint inhibitor for the use of any one of the preceding items, wherein
   (a) the first binding domain comprises a VH region as depicted in SEQ ID NO: 14 and a VL region as depicted in SEQ ID NO: 15, wherein said first binding domain is a Fab;
   (b) the second binding domain comprises a VH region as depicted in SEQ ID NO: 61 and a VL region as depicted in SEQ ID NO: 62, wherein said second binding domain is a scFv; and
   (c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.
30. The bispecific antibody construct, taxane, and/or immune checkpoint inhibitor for the use of any one of the preceding items, wherein the antibody construct comprises or consists of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.
31. The bispecific antibody construct, taxane, and/or immune checkpoint inhibitor for the use of any one of the preceding items, wherein the antibody construct comprises or consists of amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.
32. The bispecific antibody construct, taxane, and/or immune checkpoint inhibitor for the use of any one of the preceding items, wherein the bispecific antibody construct is AFM24.
33. The bispecific antibody construct, taxane, and/or immune checkpoint inhibitor for the use of any one of the preceding items, wherein the at least one first binding domain of the bispecific antibody construct comprises means for specifically binding CD16A and the at least one second binding domain of the bispecific antibody construct comprises means for specifically binding EGFR.
34. The bispecific antibody construct, taxane, and/or immune checkpoint inhibitor for the use of any one of the preceding items, wherein the bispecific antibody construct comprises means for specifically binding CD16A and EGFR.
35. The bispecific antibody construct, taxane, and/or immune checkpoint inhibitor for the use of any one of the preceding items, wherein the taxane is selected from the group consisting of docetaxel, paclitaxel, cabazitaxel.
36. The bispecific antibody construct, taxane, and/or immune checkpoint inhibitor for the use of any one of the preceding items, wherein the taxane is docetaxel.
37. The bispecific antibody construct, taxane, and/or immune checkpoint inhibitor for the use of any one of the preceding items, wherein the immune checkpoint inhibitor is a PD-L1 or PD-1 inhibitor.
38. The bispecific antibody construct, taxane, and/or immune checkpoint inhibitor for the use of any one of the preceding items, wherein the immune checkpoint inhibitor is an anti-PD-1 antibody or an anti-PD-L1 antibody.
39. The bispecific antibody construct, taxane, and/or immune checkpoint inhibitor for the use of any one of the preceding items, wherein the immune checkpoint inhibitor is an anti-PD-L1 antibody.
40. The bispecific antibody construct, taxane, and/or immune checkpoint inhibitor for the use of any one of the preceding items, wherein the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301, wherein the immune checkpoint inhibitors nivolumab atezolizumab and pembrolizumab are preferred and wherein atezolizumab is most preferred.
41. The bispecific antibody construct, taxane, and/or immune checkpoint inhibitor for the use of any one of the preceding items, wherein the immune checkpoint inhibitor is atezolizumab.
42. The bispecific antibody construct, taxane, and/or immune checkpoint inhibitor for the use of any one of the preceding items, wherein the immune checkpoint inhibitor is an antibody comprising means for specifically binding PD-L1.
43. The bispecific antibody construct, taxane, and/or immune checkpoint inhibitor for the use of any one of the preceding items, wherein the bispecific antibody construct is administered to the subject once every about 6 to about 8 days, or once every about 7 days.
44. The bispecific antibody construct, taxane, and/or immune checkpoint inhibitor for the use of any one of the preceding items, wherein the bispecific antibody construct is administered at a dose in the range of about 300 to about 800 mg, about 350 to about 700 mg, about 400 to about 550 mg, about 420 to about 530 mg, about 450 to about 500 mg, or about 480 mg.
45. The bispecific antibody construct, taxane, and/or immune checkpoint inhibitor for the use of any one of the preceding items, wherein the taxane is administered to the subject once every about 2 to about 4 weeks, once every about 3 weeks, once every about 19 to about 23 days, once every about 20 to about 22 days, or once every about 21 days.
46. The bispecific antibody construct, taxane, and/or immune checkpoint inhibitor for the use of any one of the preceding items, wherein the taxane is administered at a dose in the range of about 60 to about 90 mg/m², about 70 to about 80 mg/m², about 72 to about 78 mg/m², or about 75 mg/m².
47. The bispecific antibody construct, taxane, and/or immune checkpoint inhibitor for the use of any one of the preceding items, wherein the immune checkpoint inhibitor is administered to the subject once every about 2 to about 4 weeks, once every about 3 weeks, once every about 19 to about 23 days, once every about 20 to about 22 days, or once every about 21 days.
48. The bispecific antibody construct, taxane, and/or immune checkpoint inhibitor for the use of any one of the preceding items, wherein the immune checkpoint inhibitor is administered at a dose in the range of about 1000 to about 1400 mg, about 1100 to about 1300 mg, about 1150 to about 1250 mg, or about 1200 mg.
49. The bispecific antibody construct, taxane, and/or immune checkpoint inhibitor for the use of any one of the preceding items, wherein a cycle of administering consists of administering the bispecific antibody construct molecule 3 times, administering the taxane 1 time, and administering the immune checkpoint inhibitor 1 time, and treatment of the subject is carried out for at least one cycle.
50. The bispecific antibody construct, taxane, and/or immune checkpoint inhibitor for the use of any one of the preceding items, wherein a cycle of administering is about 3 weeks.
51. The bispecific antibody construct, taxane, and/or immune checkpoint inhibitor for the use of any one of the preceding items, wherein administering of the taxane and administering of the immune checkpoint inhibitor are separated by a time period of at least about 5 days, at least about 6 days or at least about 7 days.
52. The bispecific antibody construct, taxane, and/or immune checkpoint inhibitor for the use of any one of the preceding items, wherein the taxane is administered in the first week of each cycle.
53. The bispecific antibody construct, taxane, and/or immune checkpoint inhibitor for the use of any one of the preceding items, wherein a dose the taxane and a dose of the bispecific antibody construct are administered to the subject at the same time, or about the same time.
54. The bispecific antibody construct, taxane, and/or immune checkpoint inhibitor for the use of any one of the preceding items, wherein a dose the taxane is concurrently administered with a dose of the bispecific antibody construct.
55. The bispecific antibody construct, taxane, and/or immune checkpoint inhibitor for the use of any one of the preceding items, wherein a dose of the bispecific antibody molecule and/or a dose the taxane are administered as split-day dosing on two consecutive days.
56. The bispecific antibody construct, taxane, and/or immune checkpoint inhibitor for the use of any one of the preceding items, wherein the first dose of the bispecific antibody molecule and the dose of taxane during the first cycle are administered as split-day dosing on two consecutive days.
57. The bispecific antibody construct, taxane, and/or immune checkpoint inhibitor for the use of any one of the preceding items, wherein the immune checkpoint inhibitor is administered in the second week of each cycle.
58. The bispecific antibody construct, taxane, and/or immune checkpoint inhibitor for the use of any one of the preceding items, wherein a dose the immune checkpoint inhibitor and a dose of the bispecific antibody construct are administered to the subject at the same time, or about the same time.
59. The bispecific antibody construct, taxane, and/or immune checkpoint inhibitor for the use of any one of the preceding items, wherein a dose the immune checkpoint inhibitor is concurrently administered with a dose of the bispecific antibody construct.
60. The bispecific antibody construct, taxane, and/or immune checkpoint inhibitor for the use of any one of the preceding items, wherein a lead-in dose of the bispecific antibody construct is administered to the subject about 5 to about 8 days or about 6 to about 7 days prior to a first cycle of administration.
61. The bispecific antibody construct, taxane, and/or immune checkpoint inhibitor for the use of any one of the preceding items, wherein the lead-in dose is in the range of about 400 to about 550 mg, about 420 to about 530 mg, about 450 to about 500 mg, or about 480 mg.
62. The bispecific antibody construct, taxane, and/or immune checkpoint inhibitor for the use of any one of the preceding items, wherein the lead-in dose is administered as a split-dose on two consecutive days.
63. The bispecific antibody construct, taxane, and/or immune checkpoint inhibitor for the use of any one of the preceding items, wherein the bispecific antibody construct, the taxane, and/or the immune checkpoint inhibitor are administered intravenously.
64. The bispecific antibody construct, taxane, and/or immune checkpoint inhibitor for the use of any one of the preceding items wherein administering is carried out for a period of time in the range of 2 weeks to 2 years, or 1 month to 18 months, or 3 months to 12 months.
65. The bispecific antibody construct, taxane, and/or immune checkpoint inhibitor for the use of any one of the preceding items, wherein the method comprises assessing tumor regression after each cycle of treatment.
66. The bispecific antibody construct, taxane, and/or immune checkpoint inhibitor for the use of any one of the preceding items, wherein treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.
67. The bispecific antibody construct, taxane, and/or immune checkpoint inhibitor for the use of any one of the preceding items, wherein treating reduces the number of M2 macrophages in the tumor microenvironment as compared to a treatment regimen comprising the administration of the bispecific antibody molecule and the immune checkpoint inhibitor, but without the administration of a taxane.
68. The bispecific antibody construct, taxane, and/or immune checkpoint inhibitor for the use of any one of the preceding items, wherein treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a taxane.
69. The bispecific antibody construct, taxane, and/or immune checkpoint inhibitor for the use of any one of the preceding items, wherein treating repolarizes M2 macrophages in the tumor microenvironment to M1 macrophages, wherein the M2 macrophages are associated with the treatment of a tumor with the bispecific antibody construct.
70. A pharmaceutical composition comprising a taxane and a bispecific antibody construct that comprises at least one first binding domain capable of specifically binding CD16A and at least one second binding domain capable of specifically binding EGFR.
71. The pharmaceutical composition of item 70, wherein the taxane is present in an amount (by weight) that is in the range of about 5 to about 8 times lower than an amount of the bispecific antibody construct.
72. The pharmaceutical composition of item 70 or 71, wherein the composition is in liquid form and configured for IV administration.
73. The pharmaceutical composition of any of items 70-72, comprising one or more excipients.
74. The pharmaceutical composition of any one of items 70-73 for use in a method of treating a tumor in a subject.
75. The pharmaceutical composition for the use of item 74, wherein the use is in a method of treating as defined in any one of items 1-69.
76. A combination comprising a taxane, a bispecific antibody construct that comprises at least one first binding domain capable of specifically binding CD16A and at least one second binding domain capable of specifically binding EGFR, and optionally an immune checkpoint inhibitor.
77. The combination of item 76 for use in a method of treating a tumor in a subject.
78. The combination for the use of item 77, wherein the use is in a method of treating as defined in any one of items 1-69.
79. A taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of M2 macrophages in a tumor microenvironment associated with the treatment of a tumor with a bispecific antibody construct and optionally an immune checkpoint inhibitor.
80. The taxane, bispecific antibody construct, or combination for the use of item 79, wherein the use is in a method of treating as defined in any one of items 1-69.
81. A taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor.
82. The taxane, bispecific antibody construct, or combination for the use of item 81, wherein the use is in a method of treating as defined in any one of items 1-69.
83. A taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of repolarizing M2 macrophages in the tumor microenvironment to M1 macrophages, wherein the M2 macrophages are associated with the treatment of a tumor with a bispecific antibody construct and optionally an immune checkpoint inhibitor.
84. The taxane, bispecific antibody construct, or combination for the use of item 83, wherein the use is in a method of treating as defined in any one of items 1-69.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 10%, preferably within 5%, more preferably within 2%, even more preferably within 1% of a given value or range (plus (+) or minus (-)). It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein, any of the terms "comprising", "consisting essentially of" and "consisting of" may be replaced with either of the other two terms. For example, when disclosure uses the term "comprising", the disclosure also encompasses the replacement of the term "comprising" with the terms "consisting essentially of" as well as "consisting of" and *vice versa.* E.g., the term "comprising" is meant to provide explicit support also for its replacement with "consisting essentially of" and/or "consisting of", the term "consisting essentially of" is meant to provide explicit support also for its replacement with "comprising" and/or "consisting of", and the term "consisting of" is meant to provide explicit support also for its replacement with "consisting essentially of" and "comprising". The possibility to replace terms with each other is not to be understood that these terms are synonymous. For the avoidance of doubt, the term "comprising", "consisting essentially of" or "consisting of" that is explicitly recited in the respective context is the preferred term, while its replacement with any one of the other two terms is less preferred.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

A better understanding of the present invention and of its advantages will be obtained from the following examples, offered for illustrative purposes only. The examples are not intended to limit the scope of the present invention in any way.

### EXAMPLES

### Example 1: The CD16/EGFR specific Innate Cell Engager (ICE) AFM24 causes an immune suppressive tumor microenvironment (TME)

AFM24 is a bispecific innate cell engager (ICE) that binds to CD16A expressed on NK cells and macrophages to harness the antitumor activity of the innate immune system directed towards EGFR-expressing solid tumor cells. EGFR is frequently overexpressed on a broad range of solid tumors and is associated with poor prognosis. The AFM24 mode of action, triggering antibody-dependent cellular cytotoxicity (ADCC) and phagocytosis (ADCP), is independent of the EGFR signaling cascade and might overcome acquired resistance commonly developed upon EGFR inhibitor treatment. In the present phase I dose escalation study (Study AFM24-101/NCT04259450), AFM24 was administered at increasing doses to patients with advanced/metastatic solid tumors known to express EGFR.

The tumor microenvironment (TME) is a complex ecosystem surrounding the tumor and includes various components such as immune cells, stromal cells, blood vessels and the extracellular matrix. The interaction between these components and the tumor cells are dynamic and can influence tumor progression and the response to cancer treatment. Analysis of the TME upon AFM24 monotherapy revealed an increase in immune suppressive TME components, a potential resistance mechanism towards AFM24 that could correlate with a less effective therapy.

Based on this finding, targeting immune suppressive cells within the TME, such as M2 macrophages or Tregs, might be a potential combinatorial treatment strategy to enhance treatment effectiveness of AFM24.

### Paired tumor biopsies indicate a polarization of tumor-associated macrophages towards immune-suppressive M2 macrophages within the tumor microenvironment

Needle biopsies were taken at screening and two days after the third AFM24 dose (Cycle 1 Day 24) to assess the effect of AFM24 treatment on the tumor and the tumor microenvironment. Of note, tumor biopsies provide a snapshot of highly variable and heterogenous solid tumors and capture characteristics of the tumor from the sampled tissue area.

### Immunohistochemistry

Immunohistochemistry (IHC) allows the detection of specific antigens (proteins) within the context of tissue architecture using antibodies that bind to these antigens. In this study, IHC was performed to analyze tumor content, immune cells and EGFR expression.

In total, 15 paired biopsies could be obtained from patients treated with a dose ranging from 14 mg up to the RP2D of 480 mg. The cell density (cell per mm³) of cells expressing CD68 and CD163 was determined by image analysis using Visiopharm software. The assay was not validated and exploratory.

While the overall number of macrophages (as defined with the pan macrophage marker CD68) was not strongly affected by AFM24 treatment (data not shown), immune suppressive M2 macrophages (defined by CD163 expression) increased in the biopsies of most patients. The enhanced expression of CD163 seems to be dose-related showing a greater increase in the high dose cohort (160 - 480 mg) compared to the low dose cohort (14 - 80 mg) (Figure 1.A). At the same time an increase in CD3-positive T cells in the tumor area could be detected indicating an increase in cytotoxic T cells within the tumor (data not shown). However, the cytotoxic effect of T cells might be counteracted by an increase of M2 macrophages in the tumor reducing the antitumor effect of AFM24.

### Gene expression profiling

In addition, non-spatial gene expression profiling was performed to detect changes in cellular composition and signaling in tumor biopsies which can be linked to the treatment of AFM24. Tumor biopsies of patients treated with AFM24 at doses higher than 160 mg (high dose) were analyzed by gene expression profiling using the Nanostring nCounter^{®} PanCancer Immune Profiling Panel and Tumor Signaling 360^{™} Panel, respectively.

Briefly, 250 ng of total RNA quantified using the NanoDrop^{™} 2000 (Thermo Scientific), were directly hybridized (at 65 °C for 18 hours) with the nCounter^{®} PanCancer Immune Profiling Panel and the nCounter^{®} Tumor Signaling 360TM panel following manufacturer's instructions. After solution-phase hybridization between target RNA and reporter-capture probe pairs, excess probes were washed away using a two steps magnetic bead-based purification on the nCounter^{®} Prep Station. Finally, the RNA/Probe complexes were aligned and immobilized in the cartridge for data collection. The cartridge was then transferred to the nCounter^{®} Digital Analyzer for image acquisition and counts collection.

Data analysis of gene expression data was performed using NanoString nSolver^{®} software v4.0 and nCounter^{®} Advanced Analysis module v2.0.134. Quality control was done according to default settings using nSolver software. Background correction was conducted with background thresholding by calculating the mean of negative control expression plus double of the standard deviation. The expression counts were normalized using the most stable housekeeping genes using the geNorm algorithm according to default settings. Cell type scores were calculated as the geometric mean of log2 normalized expression levels of cell type-specific genes. Pathway scores have been calculated by pathway level analysis of gene expression (PLAGE) as implemented in the nCounter^{®} Advanced Analysis module. Visualization of results has been done using R v4.3.0. The assay was not validated and exploratory.

Findings from IHC could be confirmed at gene expression level, showing an increase of *CD163* expression in tumor biopsies upon AFM24 treatment (**Figure 1****.B**). Furthermore, cell type score calculation based on M2 macrophage specific marker genes (*CD163*, *CD68* and *CD84*) demonstrated an increase of M2 macrophages in tumor biopsies of most patients (Figure 1.C), indication a polarization of tumor-associated macrophages towards M2 macrophages upon AFM24 treatment. The induction on an immune suppressive TME comes along with an increase in expression of CD56^{dim} NK cell associated genes (data not shown), indicating NK cell engagement in line with AFM24 MoA (mode of action). Furthermore, an increase in CD56^{dim} NK cells and CD8 T cell scores as well as cytotoxicity-associated genes could be observed suggesting a migration of innate and adaptive cytotoxic cells to the tumor (data not shown). However, the anti-tumoral effect of the cytotoxic immune cells might be antagonized by immune suppressive M2 macrophages and reduce AFM24 effectiveness.

Embodiments illustratively described herein may suitably be practiced in the absence of any element or elements, limitation or limitations, not specifically disclosed herein. Thus, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present embodiments have been specifically disclosed by preferred embodiments and optional features, modification and variations thereof may be resorted to by those skilled in the art, and that such modifications and variations are considered to be within the scope of this invention. Each of the narrower species and subgeneric groupings falling within the generic disclosure also forms part of the invention. This includes the generic description of the invention with a proviso or negative limitation removing any subject matter from the genus, regardless of whether or not the excised material is specifically recited herein. In addition, where features are described in terms of Markush groups, those skilled in the art will recognize that the disclosure is also thereby described in terms of any individual member or subgroup of members of the Markush group.

Equivalents: Those skilled in the art will recognize or be able to ascertain using no more than routine experimentation, many equivalents to the specific embodiments of the invention described herein. Such equivalents are intended to be encompassed by the claims.

### SEQUENCE LISTING

| Seq ID NO | Description | Sequence |
|---|---|---|
| 1 | Linker L1 | GGSGGS |
| 2 | Linker L2 | GGS GGS GGS GGS GGS GGS |
| 3 | Linker L3 | GGSGGSGGSGGSGGSGGSGGS |
| 4 | Linker L4 | GGSGGSGGS |
| 5 | Linker L5 | GGGGS |
| 6 | Linker L6 | GGGGSGGGGS |
| 7 | Linker L7 | GGGGSGGGGSGGGGSGGGGS |
| 8 | Linker L8 | GGGGS GGGGS GGGGS GGGGS GGGGS GGGGS |
| 9 | CDR-H1 CD16A (LSIV21) | GYTFTSYY |
| 10 | CDR-H2 CD16A (LSIV21) | INPSGGST |
| 11 | CDR-H3 CD16A (LSIV21) | ARGSAYYYDFADY |
| 12 | CDR-L1 CD16A (LSIV21) | NIGSKN |
| | CDR-L2 CD16A (LSIV21) | QDN |
| 13 | CDR-L3 CD16A (LSIV21) | QVWDNYSVL |
| 14 | VH CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSS |
| 15 | VL CD16A (LSIV21) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 16 | scFv CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 17 | CDR-H1 CD16A (P2C-47) | GYTFTSYY |
| 18 | CDR-H2 CD16A (P2C-47) | IEPMYGST |
| 19 | CDR-H3 CD16A (P2C-47) | ARGSAYYYDFADY |
| 20 | CDR-L1 CD16A (P2C-47) | NIGSKN |
| | CDR-L2 CD16A (P2C-47) | QDN |
| 21 | CDR-L3 CD16A (P2C-47) | QVWDNYSVL |
| 22 | VH CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSS |
| 23 | VL CD16A (P2C-47) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 24 | scFv CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 25 | CDR-H1 CD16A (ABC1197) | GYTFTNYY |
| 26 | CDR-H2 CD16A (ABC1197) | INPSGGVT |
| 27 | CDR-H3 CD16A (ABC1197) | ARGSAYYYDFADY |
| 28 | CDR-L1 CD16A (ABC1197) | NIGSKS |
| | CDR-L2 CD16A (ABC1197) | QDK |
| 29 | CDR-L3 CD16A (ABC1197) | QVWDDYIVL |
| 30 | VH CD16A (ABC1197) | QVQLVQSGAEVKKPGESLKVSCKASGYTFTNYYMQWVRQAPGQGLEWMGIINPSGGVTSYA QKFQGRVTMTRDTSTSTVYMELSSLRSEDTAVYYCARGSAYYYDFADYWGQGTLVTVSS |
| 31 | VL CD16A (ABC1197) | SYVLTQPSSVSVAPGQTARITCGGNNIGSKSVHWYQQKPGQAPVLVIYQDKKRPSGIPERF SGSNSGNTATLTISGTQAMDEADYYCQVWDDYIVLFGGGTKLTVL |
| 32 | CDR-H1 CD16A (P2C-47var) | SYYMH |
| 33 | CDR-H2 CD16A (P2C-47var) | AIEPRYGSTSYAQKFQG |
| 34 | CDR-H3 CD16A (P2C-47var) | GSAYYYDFADY |
| 35 | CDR-L1 CD16A (P2C-47var) | GGHNIGSKNVH |
| 36 | CD16A CDR-L2 (P2C-47var) | ODNKRPS |
| 37 | CDR-L3 CD16A (P2C-47var) | QVWDNYNVL |
| 38 | CDR-H1 CD16A (ABC1197var) | NYYMQ |
| 39 | CDR-H2 CD16A (ABC1197var) | IINPSGGVTSYAQKFQG |
| 40 | CDR-H3 CD16A (ABC1197var) | GSAYYYDFADY |
| 41 | CDR-L1 CD16A (ABC1197var) | GGNNIGSKSVH |
| 42 | CDR-L2 CD16A (ABC1197var) | QDKKRPS |
| 43 | CDR-L3 CD16A (ABC1197var) | QVWDDYIVL |
| 44 | CD16A (P2C47var46) VH | EVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 45 | CD16A (P2C47var46) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 46 | CD16A P2C47var50 VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 47 | CD16A (P2C47var50) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 48 | CD16A P2C47var51 VH | QVQLVQSGAEVKKPGASVKVSCKASGYTFTSYYMHWVRQAPGQCLEWMGAIEPRYGSTSYA QKFQGRVTMTRDTSTSTVYMELSSLRSEDTAVYYCARGSAYYYDFADYWGQGTLVTVSS |
| 49 | CD16A (P2C47var51) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 50 | CD16A (P2C47var52) VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVS |
| 51 | CD16A (P2C47var52) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 52 | CD16A (ABC1197var8 ) VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSS |
| 53 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 54 | CD16A ABC1197var11 VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSSG |
| 55 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 56 | CDR-H1 EGFR | GGSVSSGSYY |
| 57 | CDR-H2 EGFR | IYYSGST |
| 58 | CDR-H3 EGFR | ARNPISIPAFDI |
| 59 | CDR-L1 EGFR | NIGSKS |
| | CDR-L2 EGFR | YDS |
| 60 | CDR-L3 EGFR | QVWDTSSDHVL |
| 61 | VH EGFR | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSS |
| 62 | VL EGFR | QPVLTQPPSVSVAPGKTARITCGGNNIGSKSVHWYQQKPGQAPVLVIYYDSDRPSGIPERF SGSNSGNTATLTISRVEAGDEADYYCQVWDTSSDHVLFGGGTKLTVL |
| 63 | AFM24 H-chain | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 64 | AFM24 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 65 | AFM24-2 H-chain | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 66 | AFM24-2 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 67 | AFM24_T | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSSGG SGGSGGSSYVLTQPSSVSVAPGQTATISCGGHNIGSKNVHWYQQRPGQSPVLVIYQDNKRP SGIPERFSGSNSGNTATLTISGTQAMDEADYYCQVWDNYSVLFGGGTKLTVLGGSGGSQVQ LVQSGAEVKKPGESLKVSCKASGYTFTSYYMHWVRQAPGQGLEWMGIINPSGGSTSYAQKF QGRVTMTRDTSTSTVYMELSSLRSEDTAVYYCARGSAYYYDFADYWGQGTLVTVSSGGSGG SGGSQPVLTQPPSVSVAPGKTARITCGGNNIGSKSVHWYQQKPGQAPVLVIYYDSDRPSGI PERFSGSNSGNTATLTISRVEAGDEADYYCQVWDTSSDHVLFGGGTKLTVLAAAGSHHHHH H |
| 68 | AFM24_Bi-scFv-Fc | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECSLTVDKSRWQQGNVFSCSVMHEALHNHYTQKSL SLSPGGGGGSGGGGSQVQLQESGPGLVKPSETLSLTCTVSGGSVSSGSYYWSWIRQPPGKG LEWIGYIYYSGSTNYNPSLKSRVTISVDTSKNQFSLKLSSVTAADTAVYYCARNPISIPAF DIWGQGTMVTVSSGGSGGSGGSGGSGGSGGSQPVLTQPPSVSVAPGKTARITCGGNNIGSK SVHWYQQKPGQAPVLVIYYDSDRPSGIPERFSGSNSGNTATLTISRVEAGDEADYYCQVWD TSSDHVLFGGGTKLTVL |
| 69 | Hinge - CH2 domain - CH3 domain | EPKSCDKTHTCPPCPAPEFEGGPSVFLFPPKPKDTLMISRTPEVTCVVVAVSHEDPEVKFN WYVDGVEVHNAKTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPG |
| 70 | C-terminal epitope of CD16A | SFFPPGYQ |
| 71 | human CD16A | MWQLLLPTALLLLVSAGMRTEDLPKAVVFLEPQWYRVLEKDSVTLKCQGAYSPEDNSTQWF HNESLISSQASSYFIDAATVDDSGEYRCQTNLSTLSDPVQLEVHIGWLLLQAPRWVFKEED PIHLRCHSWKNTALHKVTYLQNGKGRKYFHHNSDFYIPKATLKDSGSYFCRGLFGSKNVSS ETVNITITQGLAVSTISSFFPPGYQVSFCLVMVLLFAVDTGLYFSVKTNIRSSTRDWKDHK FKWRKDPQDK |
| 72 | Linker | GGGS |
| 73 | Hexahistidin e tag | HHHHHH |

## Claims

1. A bispecific antibody construct, a taxane, and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the taxane, and the immune checkpoint inhibitor.

2. The bispecific antibody construct, taxane, and/or immune checkpoint inhibitor for the use of claim 1, wherein the tumor is a solid tumor, preferably wherein the tumor **characterized by** epidermal growth factor receptor (EGFR) overexpression on tumor cells.

3. The bispecific antibody construct, taxane, and/or immune checkpoint inhibitor for the use of any one of the preceding claims, wherein the tumor is lung cancer, preferably wherein the tumor is non-small cell lung cancer (NSCLC).

4. The bispecific antibody construct, taxane, and/or immune checkpoint inhibitor for the use of any one of the preceding claims, wherein the first binding domain comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).

5. The bispecific antibody construct, taxane, and/or immune checkpoint inhibitor for the use of any one of the preceding claims, wherein the second binding domain comprises a VH and a VL domain of an antibody, optionally wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

6. The bispecific antibody construct, taxane, and/or immune checkpoint inhibitor for the use of any one of the preceding claims, wherein
(a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab;
(b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and optional
(c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

7. The bispecific antibody construct, taxane, and/or immune checkpoint inhibitor for the use of any one of the preceding claims, wherein the taxane is selected from the group consisting of docetaxel, paclitaxel, cabazitaxel, preferably wherein the taxane is docetaxel.

8. The bispecific antibody construct, taxane, and/or immune checkpoint inhibitor for the use of any one of the preceding claims, wherein the immune checkpoint inhibitor is a PD-L1 or PD-1 inhibitor, preferably wherein the immune checkpoint inhibitor is an anti-PD-1 antibody or an anti-PD-L1 antibody, optionally wherein the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301, preferably the immune checkpoint inhibitor is selected from the group consisting of nivolumab atezolizumab and pembrolizumab, most preferably the immune checkpoint inhibitor is atezolizumab.

9. The bispecific antibody construct, taxane, and/or immune checkpoint inhibitor for the use of any one of the preceding claims, wherein the bispecific antibody construct is AFM24, according to one of the claims 4 to 6, and wherein the taxanes is docetaxel, and wherein the immune checkpoint inhibitor is atezolizumab.

10. The bispecific antibody construct, taxane, and/or immune checkpoint inhibitor for the use of any one of the preceding claims, wherein
- the bispecific antibody construct is administered to the subject once every about 6 to about 8 days, or once every about 7 days, optionally wherein the bispecific antibody construct is administered at a dose in the range of about 300 to about 800 mg, about 350 to about 700 mg, about 400 to about 550 mg, about 420 to about 530 mg, about 450 to about 500 mg, or about 480 mg. preferably the taxane is administered to the subject once every about 2 to about 4 weeks, once every about 3 weeks, once every about 19 to about 23 days, once every about 20 to about 22 days, or once every about 21 days; and
- the taxane is administered to the subject once every about 2 to about 4 weeks, once every about 3 weeks, once every about 19 to about 23 days, once every about 20 to about 22 days, or once every about 21 days, optionally wherein the taxane is administered at a dose in the range of about 60 to about 90 mg/m2, about 70 to about 80 mg/m2, about 72 to about 78 mg/m2, or about 75 mg/m2; and/or
- the immune checkpoint inhibitor is administered to the subject once every about 2 to about 4 weeks, once every about 3 weeks, once every about 19 to about 23 days, once every about 20 to about 22 days, or once every about 21 days; optionally wherein the immune checkpoint inhibitor is administered at a dose in the range of about 1000 to about 1400 mg, about 1100 to about 1300 mg, about 1150 to about 1250 mg, or about 1200 mg.

11. The bispecific antibody construct, taxane, and/or immune checkpoint inhibitor for the use of any one of the preceding claims, wherein
- the bispecific antibody construct, preferably AFM24, is administered to the subject on day 1, 8, and 15 of each 21-day cycle at a dose of 480 mg weekly; and
- the taxane, preferably docetaxel, is administered to the subject on day 1 of each 21-day cycle at a dose of 75 mg/m2 every 3 weeks (q3w); and
- the immune checkpoint inhibitor, preferably atezolizumab, is administered to the subject on day 8 of each 21-day cycle at a dose of 1200 mg q3w;
wherein the bispecific antibody construct and the taxanes is administered to the subject on day 1 of the first and second cycle as a split day-dose on day 1 and 2 and wherein the treatment lasts up to 24 months or disease progression, optionally wherein a lead-in dose of the bispecific antibody construct, preferably AFM24, is administered to the subject as a split-day dose on days 7 and 6 prior to the first cycle in an amount of 480 mg.

12. The bispecific antibody construct, taxane, and/or immune checkpoint inhibitor for the use of any one of the preceding claims, wherein treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor, optionally wherein treating reduces the number of M2 macrophages in the tumor microenvironment as compared to a treatment regimen comprising the administration of the bispecific antibody molecule and the immune checkpoint inhibitor, but without the administration of a taxanes, optionally wherein treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a taxanes, further optionally wherein treating repolarizes M2 macrophages in the tumor microenvironment to M1 macrophages, wherein the M2 macrophages are associated with the treatment of a tumor with the bispecific antibody construct.

13. A combination comprising a taxane, a bispecific antibody construct that comprises at least one first binding domain capable of specifically binding CD16A and at least one second binding domain capable of specifically binding EGFR, and optionally an immune checkpoint inhibitor, optionally for use in a method of treating a tumor in a subject, wherein the use is optionally in a method of treating as defined in any one of claims 1-12.

14. A taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of M2 macrophages in a tumor microenvironment associated with the treatment of a tumor with a bispecific antibody construct and optionally an immune checkpoint inhibitor; optionally wherein the use is in a method of treating as defined in any one of claims 1-12.

15. A taxane, a bispecific antibody construct, or a combination of a taxane, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of repolarizing M2 macrophages in the tumor microenvironment to M1 macrophages, wherein the M2 macrophages are associated with the treatment of a tumor with a bispecific antibody construct and optionally an immune checkpoint inhibitor, optionally wherein the use is in a method of treating as defined in any one of claims 1-12.
